# DEMANDE DE BREVET EUROPEEN

(11) **EP 1 041 067 A2**
(43) Date de publication de la demande: **04.10.2000**
(21) Numéro de dépôt: 00113228.1
(22) Date de dépôt: 22.12.1994
(51) Int. Cl.: C07D 233/84, A61K 31/415, A61P 39/06

(54) **Utilisation de dérivés 2-mercapto-imidazole substitués en position 4 (ou 5) comme agents antioxydants**

(30) Priorité: 24.12.1993 FR 9315637
(62) Demande divisionnaire de: 95904589.9
(71) Demandeur: OXIS Isle of Man, Limited, Douglas 1M1 3DB, Isle of Man (GB)
(72) Inventeur: Yadan, Jean-Claude, 75011 Paris (FR); Xu, Jinshu, 94200 Ivry-sur-Seine (FR); Moutet, Marc, 92220 Bagneux (FR); Chaudiere, Jean, 94100 Saint-Maur-des-Fausses (FR)
(74) Mandataire: Portal, Gérard

(57) **Abrégé**

Des dérivés 2-mercapto-imidazole substitués en position 4 (ou 5 ) de la formule générale (I) dans laquelle R₁, R₂, R₅ à R₈ sont l'hydrogène, un alkyle inférieur en C₁-C₆, un ar(alkyle) en C₁-C₆) ou un ar(alkyle inférieur en C₁-C₆) substitué;
R₃ représente -(CH₂)ₙCOR₄ ; -(CH₂)ₙN⁺(R₅R₆R₇), X⁻ ; -CH₂CH(COR₄)N⁺(R₅R₆R⁷), X⁻
R₄ représente :
   a) -OR₈ lorsque R₃ représente -CH₂CH(COR₄)N⁺(R₅R₆R₇), X⁻;-α-aminoacide, de préférence -α-aminoacide naturel; 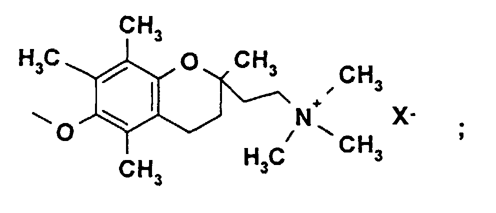
   b) lorsque au moins un parmi R₁, R₂ et R₅ à R₈ représente ar(alkyle inférieur C₁-C₆) ou ar(alkyle inférieur en C₁-C₆) substitué tel que défini, R4 peut représenter -OR₈ ; -NHR₅ ;-NHCH₂CH₂SO₃⁻,Y⁺; -NHCH₂CH₂CO₂⁻,Y⁺; -OCH₂CH₂N⁺(CH₃)₃, X⁻; 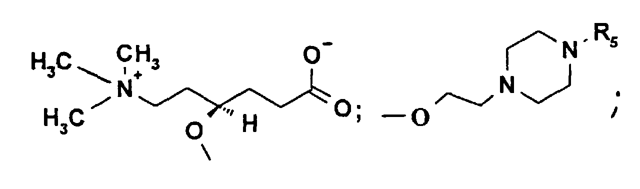 n = 1ou 2; X⁻ est un anion d'un acide acceptable en cosmétique, pharmacie ou dans l'industrie alimentaire; Y⁺ est un cation d'une base acceptable en cosmétique, pharmacie ou industrie alimentaire. Ces composés sont utiles comme agent antioxydant et comme ingrédient actif d'une composition ayant une activité antioxydante utile en cosmétique, pharmacie et industrie alimentaire.

## Description

La présente invention concerne l'utilisation de dérivés 2-mercapto-imidazole substitués en position 4 (ou 5) comme agents antioxydants ainsi que leur procédé de préparation et des compositions pharmaceutiques, cosmétiques ou alimentaires en comportant application.

### ETAT DE L'ART ANTERIEUR:

Il est décrit dans le Journal of the Chemical Society 1954, Letchworth GB, page 3287, ligne 27, un composé B-(2-mercapto-4-glyoxalinyl) propionamide qui est exclus par l'exclusion a) de la revendication de produit selon laquelle, lorsque R₄= -NHR₅, alors R₁ et R₂ ne peuvent représenter simultanément l'hydrogène.

Plusieurs dérivés 2-mercapto-imidazole substitués en position 4 ou 5 par un groupement carbonylé (cétonique, carboxylique ou amide) ont révélé une activité antioxydante (voir R.C. SMITH et coll. ; Biochem. Pharmacol. ; (1987) ; 36 ; 9 ; pages 1457-1460) et anti-inflammatoire (voir S. MAEDA et coll. ; Chem. Pharm. Bull. ; (1984) ; 32 ; pages 2536-2543).

Les propriétés antioxydantes des nouveaux dérivés 2-mercapto-imidazole, qui font l'objet de la présente invention, s'apparentent à celle de la L-(+)-ergothionéine, qui en fait partie.

La L-(+)-ergothionéine, molécule naturelle possèdant une structure 2-mercaptoimidazole, est biosynthétisée par certains champignons de l'ergot de seigle, tels *Claviceps purpurea* (voir C. TANRET; C.R. Acad. Sci.; (1909); 149; pages 222-224). La structure chimique de la L-(+)-ergothionéine est rare dans le monde vivant dans la mesure où elle est composée d'un noyau 2-mercapto-imidazole et d'une bétaine (voir G.G. SKELLERN; in "Sulfur-containing Drugs and related organic compounds: Chemistry, Biochemistry and Toxicology" L.A. DAMANI Eds.; Ellis Horwood Lim.; (1989); Vol.1, part B, Chap. 3; pages 49-89). L'homme est auxotrophe pour l'ergothionéine, qui lui est exclusivement apportée par voie alimentaire. Les concentrations physiologiques d'ergothionéine varient entre (0,1-2,0 mmolaires) dans les érythrocytes, le foie, le rein, le fluide seminal et le cristallin sans cataracte. Bien que le rôle biologique de l'ergothionéine reste incertain, ses propriétés antioxydantes sont bien documentées (voir P.E. Hartman; Meth. Enzymol.; (1990); 186; pages 310-318 et D. AKANMU et coll.; Arch. Biochem. Biophys.; (1991); 288; pages 10-16). En conditions physiologiques (concentrations et pH), elle ne réagit pas avec le peroxyde d'hydrogène H₂O₂ ni avec l'anion superoxyde O₂.- (voir D. AKANMU et coll.; Arch. Biochem. Biophys.; (1991); 288; pages 10-16). Par contre, elle réagit avec les radicaux OH- produits par radiolyse pulsée (voir M. ROUGEE et coll.; Photochem. Photobiol.; (1988); 47; pages 485-489) ou via la réaction de Fenton (voir D. AKANMU et coll.; Arch. Biochem. Biophys.; (1991); 288; pages 10-16) avec une cinétique proche de la vitesse de diffusion limite ; elle réagit avec l'acide hypochloreux HOCI empêchant ainsi l'inactivation de l'al-antitrypsine (voir D. AKANMU et coll.; Arch. Biochem. Biophys.; (1991); 288; pages 10-16) ; et inhibe la photoproduction d'oxygène singulet par quenching des états excités de photo-sensibilisateurs tels que le rose Bengal (voir S.S. SPICER et coll.; Proc. Soc. Exp. Biol. Med.; (1951); 77; page 418). De plus l'ergothionéine, comme la plupart des dérivés 2-mercapto-imidazole, forme des complexes très stables avec les métaux divalents tels que Cu⁺⁺, Hg⁺⁺, Zn⁺⁺, Co⁺⁺ et Ni⁺⁺ (voir D.P. HANLON; J. Med. Chem.; (1971); 14; page 1084 et N. MOTOHASHI et coll.; Chem. Pharm. Bull.; (1974); 22; pages 654-657). Contrairement à de nombreux alkylmercaptans RSH tels que par exemple le glutathion ou la cystéine, l'ergothionéine ne stimule pas la peroxydation des acides gras poly-insaturés en présence de sels métalliques (Fe⁺⁺) (voir D. AKANMU et coll.; Arch. Biochem. Biophys.; (1991); 288; pages 10-16), ce qui est en accord avec ses propriétés de chélateur inactivant ainsi qu'avec sa structure thione majoritaire. Un autre des avantages que présente l'utilisation d'antioxydants de structure 2-mercapto-imidazole est leur très grande stabilité en solution aqueuse aérée. En effet, l'équilibre tautomérique des dérivés 2-mercapto-imidazole étant totalement déplacé vers la forme thione en solution (voir E. BOJARSKA-OLEJNIK et coll.; Mag. Res. Chem.; (1985); 23; pages 166-169), l'atome de soufre du noyau 2-mercapto-imidazole ne réagit pratiquement pas avec l'oxygène dissous. Encore un autre des avantages que présente l'utilisation d'antioxydants de structure 2-mercapto-imidazole est que leurs disulfures ne sont pas stables en présence d'autre mercaptan tel que par exemple la cystéine, la cystéamine, le glutathion ou l'acide lipoique.

A des concentrations micromolaires, l'ergothionéine ainsi que certains dérivés 2-mercapto-imidazole, inhibent, de manière efficace, la formation de methémoglobine à partir d'oxyhémoglobine incubée en présence de nitrite de sodium in vitro (voir R.C. SMITH et coll.; Biochem. Pharmacol.; (1987); 36; 9; pages 1457-1460 et R.A. MORTENSEN; Arch. Biochem. Biophys.; (1953); 46; pages 241-243). Elle réduit les formes ferryl des protéines héminiques qui sont produites en présence de peroxyde d'hydrogène H₂O₂ à pH physiologique (voir A. ARDUINI et coll.; Arch. Biochem. Biophys.; (1990); 281; pages 41-43). La réduction rapide de ferrylmyoglobine (Mb^{IV}) pourrait être un mécanisme essentiel de protection du tissu musculaire, en général, et du tissu cardiaque, en particulier, par l'ergothionéine, lors d'un stress oxydant et en particulier lors d'une reperfusion post-ischémique. Il a été montré sur un modèle de reperfusion post-ischémique de coeur de rat isolé, qu'après 15 min. d'ischémie, l'ergothionéine (100 pmolaires) limitait l'importance de la nécrose cellulaire évaluée par la mesure de l'activité lactate-deshydrogénase de l'effluent (voir A. ARDUINI et coll.; Arch. Biochem. Biophys.; (1990); 281; pages 41-43).

Sur le plan chimique, l'accès aux dérivés 2-mercapto-imidazole a été réalisé selon deux voies principales, à savoir:
* génération du noyau 2-mercapto-imidazole soit par réaction d'un dérivé α-amino-cétonique avec le thiocyanate de potassium (voir S. MAEDA et coll.; Chem. Pharm. Bull.; (1984); 32; pages 2536-2543 et Y. ISOMURA et coll.; Chem. Pharm. Bull.; (1984); 32; pages 152-165 ainsi que J. FERNANDEZ-BOLANOS et coll.; Anales de Quimica; (1974); 70; pages 94-95)); soit par réaction d'un dérivé α-halogèno-cétonique avec la thiourée ou l'un de ses dérivés ;
* introduction du soufre, en position 2, sur un noyau imidazole soit par addition nucléophile d'un dérivé soufré sur un noyau imidazole électrophile (voir S. ITO; J. Org. Chem.; (1985); 50; pages 3636-3638), soit par addition électrophile de soufre sur un noyau imidazole nucléophile (voir B.L. BENAC et coll.; Org. Synthesis; coll.vol. VII; pages 195-196).

Il est à noter que dans le document de Smith précité, publié dans Biochem. Pharm. (1987), Vol. 36, No. 9, pages 1457-1460, on décrit des dérivés 2-imidazoles ayant une activité anti-oxydante mais dont tous les composés 2-mercaptoimidazole ont obligatoirement deux substituants, respectivement en position 4 et 5 du cycle imidazole, de type ester, amino ou amide non-substitué ou substitué et directement lié à l'une des positions du cycle, contrairement à la présente invention et où les analogues les plus proches sont fixés par au moins un groupement CH2.

Le document US-A-4,898,878 décrit des composés antioxydants thérapeutiques comportant deux substituants en position 4 et 5 du cycle imidazole, un premier substituant de type SR₅ et un deuxième substituent de type 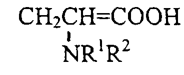 différents de ceux de l'invention.

L'invention a également pour but de résoudre le nouveau problème technique énoncé ci-dessus selon une solution fournissant également un procédé de préparation de ces composés aisé à mettre en oeuvre, avec de bons rendements.

Le problème technique énoncé ci-dessus est résolu pour la première fois, d'une manière simultanée par la présente invention selon une solution simple, avec un procédé de préparation relativement aisé à mettre en oeuvre et fournissant de bons rendements, ainsi qu'un produit d'une extrême pureté, en particulier optique.

En effet, l'invention propose une nouvelle méthode d'introduction du soufre, en position 2, sur un noyau imidazole dont le mécanisme s'apparenterait à un mécanisme du type Addition Nucléophile-Ouverture-Refermeture du Cycle (ANORC). Les conditions opératoires de cette nouvelle méthode sont suffisamment douces pour permettre de maintenir l'intégrité d'un centre chiral, en particulier celui du carbone a d'un amino-acide.

Ainsi, selon un premier aspect, la présente invention concerne l'utilisation de dérivés 2-mercapto-imidazole substitués en position 4 (ou 5) de formule chimique générale (I) suivante : 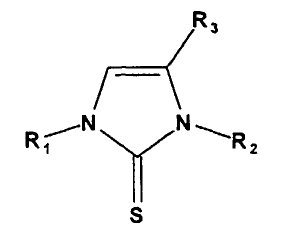 dans laquelle :
R₁ représente l'hydrogène, un alkyle inférieur, en C₁-C₆, un groupement ar(alkyle en C₁-C₆) ou ar(alkyle C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₂ représente l'hydrogène, un alkyle inférieur, en C₁-C₆ un groupement ar(alkyle en C₁-C₆) ou ar(alkyle C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
   sachant qu'au moins un parmi R₁ et R₂ représente l'hydrogène;
R₃ représente -(CH₂)ₙCOR₄ ; -(CH₂)ₙN⁺(R₅R₆R₇), X⁻ ; -CH₂CH(COR₄)N⁺(R₅R₆R₇), X⁻;
R₄ représente :
   a) -OR₈ lorsque R₃ représente -CH₂CH(COR₄)N⁺(R₅R₆R₇), X⁻;-α-aminoacide, de préférence -α-aminoacide naturel; 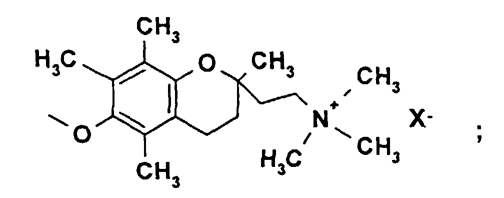
   b) lorsque au moins un parmi R₁, R₂ et R₅ à R₈ représente ar(alkyle inférieur en C₁-C₆) ou ar(alkyle inférieur en C₁-C₆) substitué tel que défini, R₄ peut représenter -OR₈ ; -NHR₅ ; -NHCH₂CH₂SO₃⁻,Y⁺; -NHCH₂CH₂CO₂⁻,Y⁺; -OCH₂CH₂N⁺(CH₃)₃, X⁻; 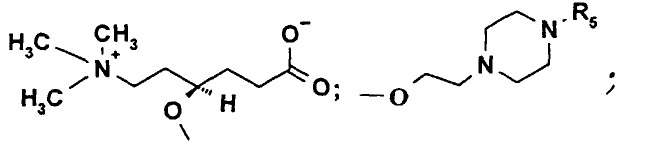
R₅ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₆ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₇ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₈ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
   n= 1 ou 2
   X⁻ représente un anion d'un acide cosmétiquement, pharmaceutiquement ou alimentairement acceptable,
   Y⁺ représente un cation d'une base cosmétiquement, pharmaceutiquement ou alimentairement acceptable,
comme agents antioxydants.

Dans le cadre de la description et des revendications :
- par groupement alkyle, ou alkyle inférieur, alkoxy, ou alkoxy inférieur, acyle ou amino acyle, carboxyle, on entend de préférence des groupements linéaires ou ramifiés comprenant 1 à 6 atomes de carbone ;
- lorsque R₈ représente un atome d'hydrogène, l'invention couvre également les sels d'addition à une base pharmaceutiquement, cosmétiquement ou alimentairement acceptable des composés de formule (I) précités ;
- lorsque R₅, R₆ ou R₇ représente un atome d'hydrogène, l'invention couvre également les sels d'addition à un acide pharmaceutiquement, cosmétiquement ou alimentairement acceptable des composés de formule (I) précités.

Parmi les acides pharmaceutiquement, cosmétiquement ou alimentairement acceptables, on peut citer, à titre non limitatif, les acides chlorhydrique, bromhydrique, iodhydrique, sulfurique, tartrique, méthanesulfonique, trifluorométhanesulfonique, etc...

Parmi les bases pharmaceutiquement, cosmétiquement et alimentairement aceptables, que l'on peut le cas échéant ajouter aux composés pour lesquels R₈ est un atome d'hydrogène pour obtenir un sel, on peut citer, à titre non limitatif, les hydroxydes de sodium, de potassium, les carbonates de métaux alcalins ou alcalino-terreux ou des bases organiques comme la triéthylamine ou l'arginine, etc...

Selon un mode de réalisation avantageux, la présente invention concerne l'utilisation des composés précités de formule (I) pour la fabrication d'une composition pharmaceutique à activité antioxydante, en particulier pour le traitement d'une pathologie impliquant un stress oxydant associé à une surproduction de radicaux libres oxydants et/ou à une décompartimentation intracellulaire du pool de certains métaux de transition tels que le fer, le cuivre ou le manganèse.

Selon un autre mode de réalisation avantageux, la présente invention concerne l'utilisation des composés précités de formule (I) pour la fabrication d'une composition pharmaceutique pour la prévention des altérations tissulaires induites par une ischémie ou une reperfusion post-ischémique, et en particulier la prévention d'infarctus du myocarde, et la prévention des arythmies cardiaques postischémiques source de fibrillation ventriculaire ; pour la prévention des altérations tissulaires telles qu'oedème, nécroses et fibroses associées à une surproduction de radicaux libres, comprenant notamment le traitement d'intoxications par les xénobiotiques tels que par exemple le paraquat, le diquat, les anthracyclines ou les nitrofurannes ; ou les pathologies associées à un stress oxydant au niveau érythrocytaire, en particulier l'anémie falciforme, la thalassémie, les maladies de déficience en glucose-6-phosphate déshydrogénase et la malaria ; ou pour la protection contre l'irradiation par des rayons ionisants X ou gamma, ainsi que les rayons UV ; ou la protection dans des milieux de conservation de greffons, comme par exemple le coeur, le foie, le rein ou le poumon, en transplantation d'organes.

Selon un autre mode de réalisation avantageux, la présente invention concerne l'utilisation des composés de formule (I) précitée pour la fabrication de compositions cosmétiques à activité antioxydante, en particulier pour la protection contre les rayons UV.

Selon un autre mode de réalisation avantageux, la présente invention concerne l'utilisation des composés de formule (I) précitée pour la fabrication de compositions alimentaires à activité antioxydante.

Selon un autre mode de réalisation avantageux, le 2-mercaptoimidazole de formule (I) précitée est présent en une quantité comprise entre 0,1 et 5 % en poids par rapport au poids total de la composition finale, de préférence entre 0,1 et 1 % en poids.

Selon un autre mode de réalisation avantageux, la présente invention concerne l'utilisation de la composition sous forme de dose unitaire pouvant comprendre de 1 à 500 mg, de dérivé 2-mercapto-imidazole, éventuellement dans un excipient, véhicule ou support pharmaceutiquement acceptable.

Selon un deuxième aspect, la présente invention fournit également une composition pharmaceutique, cosmétique ou alimentaire, en particulier à activité antioxydante, caractérisée en ce qu'elle comprend, à titre d'ingrédient actif, au moins un composé 2-mercapto-imidazole de formule générale (I) suivante : 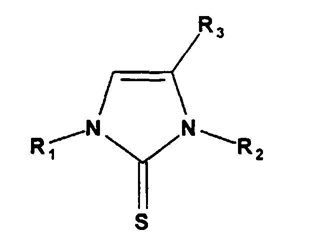 dans laquelle :
R₁ à R₈ sont tels que définis ci-dessus selon le premier aspect ou à la revendication 1;
n= 1 ou 2
X⁻ représente un anion d'un acide cosmétiquement, pharmaceutiquement ou alimentairement acceptable,
Y⁺ représente un cation d'une base cosmétiquement, pharmaceutiquement ou alimentairement acceptable,
éventuellement dans un excipient, support ou véhicule pharmaceutiquement, cosmétiquement ou alimentairement acceptable.

D'autres modes de réalisation particuliers de cette composition apparaissent clairement à partir de la description précédente et apparaitront aussi clairement à un homme de l'art à partir de la description suivante, incluant les exemples.

Selon un troisième aspect, la présente invention couvre également un procédé de fabrication de 2-mercapto-imidazole de formule générale (I) suivante : 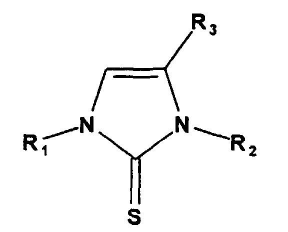 dans laquelle :
R₁ à R₈ sont tels que définis précédemment selon le premier aspect ou le deuxième aspect, ou à la revendication 1;
n= 1 ou 2
X⁻ représente un anion d'un acide cosmétiquement, pharmaceutiquement ou alimentairement acceptable,
Y⁺ représente un cation d'une base cosmétiquement, pharmaceutiquement ou alimentairement acceptable,
caractérisé en ce qu'il comprend les étapes essentielles suivantes :
a) préparer ou utiliser un dérivé imidazole substitué en position 4 (ou 5) éventuellement protégé; si nécessaire optiquement actif ;
b) traiter ce dérivé imidazole par un halogénothionoformiate d'alkyle, d'alkènyle ou d'aryle, en milieu basique dans un solvant polaire ;
c) puis, selon les cas :

i - pour la préparation de composés de formule (I) précédente, dans lesquels R³ présente les définitions précitées à la condition que R⁵, R⁶ et R⁷ ne sont pas tous simultanément autres que l'hydrogène, hydrolyser en milieu basique, ou en milieu acide en présence d'un piégeur de carbocation ;
ii - pour la préparation des composés de formule (I) précitée dans lesquels R⁵, R⁶ et R⁷ sont simultanément autres que l'hydrogène, protéger le substituant soufré, puis transformer le composé protégé en un composé trialkylammonium et hydrolyser en milieu basique, ou en milieu acide en présence d'un piégeur de carbocation, de préférence en milieu acide en présence d'un piégeur de carbocation si l'on veut préserver l'activité optique d'un centre chiral prééxistant.

Selon un mode de réalisation avantageux de ce procédé, celui-ci est caractérisé en ce que le piégeur de carbocation précité est un mercaptan, de préférence un alkyle ou un aryle mercaptan, et encore mieux l'acide (3-mercaptopropionique.

Selon encore un autre mode de réalisation avantageux, la base précitée est de préférence le bicarbonate de sodium, une amine ou une alkylamine telle que par exemple la diéthylamine ou la triéthylamine.

Selon un autre mode de réalisation particulier, le solvant polaire précité peut être choisi parmi un solvant éthéré comme par exemple l'éther éthylique ou le tétrahydrofuranne, ou un alcool comme par exemple le méthanol.

Selon encore un autre mode de réalisation avantageux, l'hydrolyse basique précitée est réalisée avec une base inorganique, telle que par exemple la soude ou la lithine, ou organique telle qu'une amine, une alkylamine, en particulier la diéthyle ou la triéthylamine, en particulier en solution dans un solvant polaire, de préférence comprenant un mélange eau/alcool, en particulier le méthanol.

Selon un autre mode de réalisation avantageux, l'hydrolyse acide précitée est réalisée à l'aide d'une solution d'acide fort concentré, à pH inférieur à 2, en présence d'un piégeur de carbocation, en particulier un mercaptan, de préférence en large excès.

Selon un autre mode de réalisation avantageux, la protection des substituants soufrés précités est effectuée au moyen d'un halogénoformiate, de préférence un halogénoformiate d'alkyle ou de phényle, par exemple le chloroformiate d'éthyle ou de phényle ; la transformation en composé trialkylammonium est réalisée au moyen d'un agent alkylant comme par exemple un halogénure ou sulfate d'alkyle, en particulier l'iodure de méthyle ou le sulfate de diméthyle.

Selon une autre caractéristique particulièrement avantageuse, dans le cadre de la préparation d'un dérivé final optiquement actif, on utilise un composé de départ optiquement actif qui est hydrolysé au moyen d'une solution acide concentrée à pH inférieur à 2 et en présence d'un piégeur de carbocation, de préférence un mercaptan, en large excès.

Selon un quatrième aspect, la présente invention fournit de nouveaux dérivés 2-mercapto-imidazole substitués en position 4 (ou 5) de la formule (I) générale suivante : 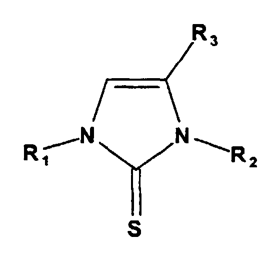 dans laquelle :
R₁, R₂ à R₈ sont tels que définis à la revendication 1 et n=2.

Comme il a été dit précédemment, les composés 2-mercaptoimidazole substitués en position 4 (ou 5) de la formule (I) précitée constituent de précieux agents antioxydants.

Dans ce cadre, ils constituent de précieux agents actifs lors d'une application thérapeutique.

D'une manière générale, les applications thérapeutiques des composés de structure générale I incluent toutes les pathologies impliquant un stress oxydant associées à une surproduction de radicaux libres oxydants et/ou à une décompartimentation intracellulaire du pool de certains métaux de transition tels que le fer, le cuivre ou le manganèse par exemple.

Plus spécifiquement, elles incluent:
* la prévention des altérations tissulaires induites par une ischémie et/ou une reperfusion post-ischémique, et en particulier la prévention d'infarctus du myocarde, et celle des arythmies cardiaques post-ischémiques source de fibrillation ventriculaire ;
* la prévention des altérations tissulaires telles qu'oedème, nécroses et fibroses associées à une surproduction de radicaux libres : cette application inclue notamment le traitement d'intoxications par des xénobiotiques tels que par exemple le paraquat, le diquat, les anthracyclines ou les nitrofuranes ;
* les pathologies associées à un stress oxydant au niveau érythrocytaire, en particulier l'anémie falciforme, la thalassémie, les maladies de déficience en glucose-6-phosphate deshydrogénase et la malaria ;
* la protection contre l'irradiation par des rayons ionisants X ou gamma, ainsi que par des rayons UV ;
* la protection, dans des milieux de conservation, de greffons, comme par exemple le coeur, le foie, le rein ou le poumon, en transplantation d'organes.

Dans cette application thérapeutique, les dérivés de formule (I) précitée de l'invention seront avantageusement présentés sous forme de dose unitaire pouvant comprendre de 1 à 500 mg de dérivés 2-mercapto-imidazole, éventuellement dans un excipient, véhicule ou support pharmaceutiquement acceptable.

De tels excipients, véhicules ou supports pharmaceutiquement acceptables sont bien connus de l'homme de l'art et ne sont donc pas détaillés ici.

Les activités antioxydante et thérapeutique ou pharmacologique des dérivés 2-mercapto-imidazole substitués en position 4 (ou 5) de la formule (I) précitée ont été mises en évidence selon des tests sûrs et fiables bien reconnus par l'homme de l'art et qui comprennent :
a) le test de réduction de la ferrylmyoglobine ;
b) le test de prévention de l'inactivation de la glutathion peroxydase par l'acide hypochloreux ;
c) le test de prévention de l'inactivation de la glucose-6-phosphate Deshydrogénase par le système Cu(II)/ascorbate/O₂ ;
d) le test de prévention de la dégradation de l'ADN par le système Fe(II)-citrate/H₂O₂/ascorbate ;
e) le test d'inhibition de la nécrose cardiaque induite par une période d'ischémie-reperfusion;
f) le test de protection de la fonction mécanique (ventriculaire) d'un coeur soumis à une période d'ischémie.

Compte tenu de ces activités antioxydante et thérapeutique/ pharmacologique, les dérivés 2-mercapto-imidazole substitués en position 4 (ou 5) de la formule (I) générale précitée permettent de réaliser les applications thérapeutiques précédemment énoncées et plus particulièrement :
* la prévention des altérations tissulaires induites par une ischémie et/ou une reperfusion post-ischémique, et en particulier la prévention d'infarctus du myocarde et celles des arythmies cardiaques post-ischémiques qui sont source de fibrillation ventriculaire ;
* la prévention des altérations tissulaires telles qu'oedème, nécroses et fibroses associées à une surproduction de radicaux libres: cette application inclue notamment le traitement d'intoxications par des xénobiotiques tels que par exemple le paraquat, le diquat, les anthracyclines ou les nitrofurannes ;
* les pathologies associées à un stress oxydant au niveau érythrocytaire, en particulier l'anémie falciforme, la thalassémie, les maladies de déficience en glucose-6-phosphate deshydrogénase et la malaria ;
* la protection contre l'irradiation par des rayons ionisants X ou gamma, ainsi que par des rayons UV ;
* la protection, dans des milieux de conservation, de greffons, comme par exemple le coeur, le foie, le rein ou le poumon, en transplantation d'organes.

D'autres buts, caractéristiques et avantages de l'invention apparaitront clairement à la lumière de la description explicative qui va suivre faite en référence à divers exemples non limitatifs donnés simplement à titre d'illustration et qui ne sauraient donc en aucune façon limiter la portée de l'invention. Dans les exemples, tous les pourcentages sont donnés en poids sauf indication contraire.

Dans les figures annexées,

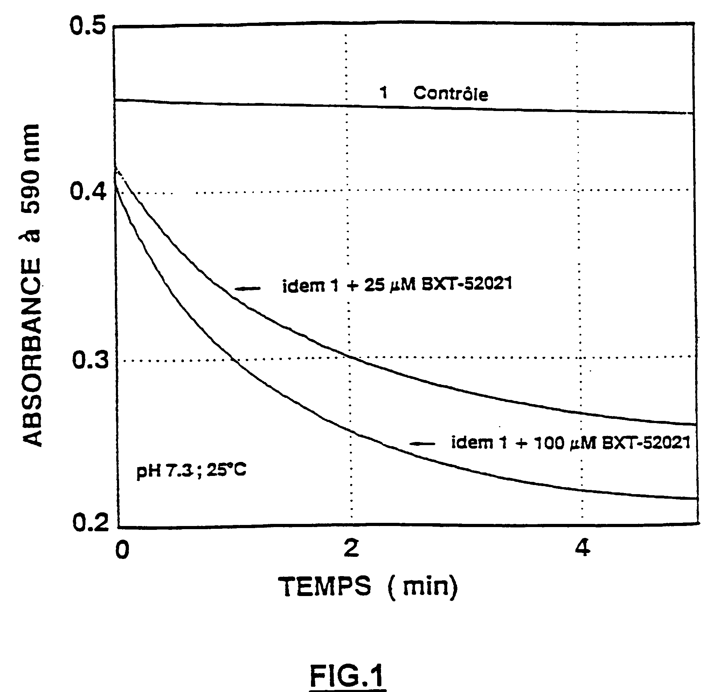

- la figure 1 représente les courbes de réduction de la ferrylmyoglobine avec en abscisse le temps exprimé en minute et en ordonnée l'absorbance à 590 nm ;

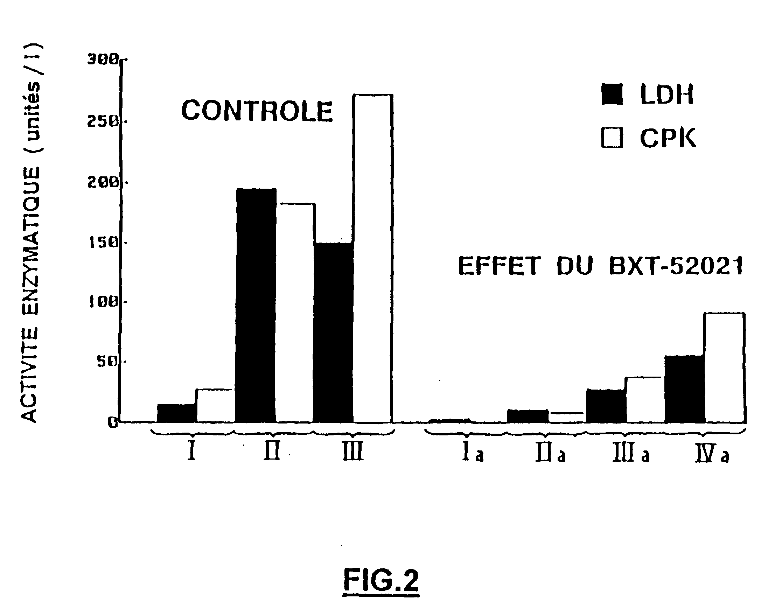

- la figure 2 représente l'effet du composé de l'invention référence BXT-52021 sur la libération de la lactate deshydrogènase (LDH) et la créatine phosphokinase (CPK) dans un coeur de rat isolé et perfusé, soumis à une ischémie-reperfusion, avec en abscisse les périodes respectivement pour le contrôle et le composé de l'invention BXT-52021 pour la LDH en noir et la CPK en blanc, avec en ordonnée l'activité enzymatique obtenue en unité/liltre. Pour le contrôle, les chiffres I, II et III signifient respectivement :

- I = fin de stabilisation ;
- II = 5 min après reperfusion ;
- III = 15 min après reperfusion.

Pour le composé BXT-52021, les périodes IA ; IIA ; IIIA ; IVA signifient respectivement :
- IA : fin de stabilisation ;
- IIA = 12 min après la perfusion par le composé BXT-52021 (2 µM) ;
- IIIA = 5 min après reperfusion post-ischémique avec BXT-52021 (2 µM) ;
- IVA : 15 min après reperfusion post-ischémique avec BXT-52021 (2µM).

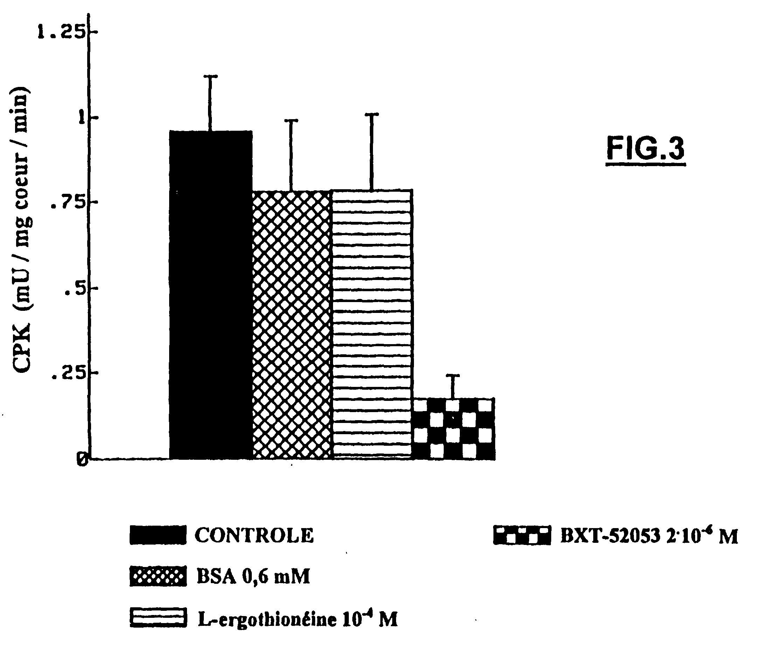

- la figure 3 représente l'effet du composé de l'invention référence BXT 52053 sur la libération de la créatine phosphokinase (CPK) dans un coeur de rat, isolé et perfusé, soumis à une ischémie-reperfusion, avec en ordonnée la quantité totale de CPK libérée (exprimée en milli-unités d'activité enzymatique par milligramme de coeur et par minute). L'effet du composé BXT 52053 est en outre comparé à celui de l'albumine (BSA) et de la L-(+)-ergothionéine.

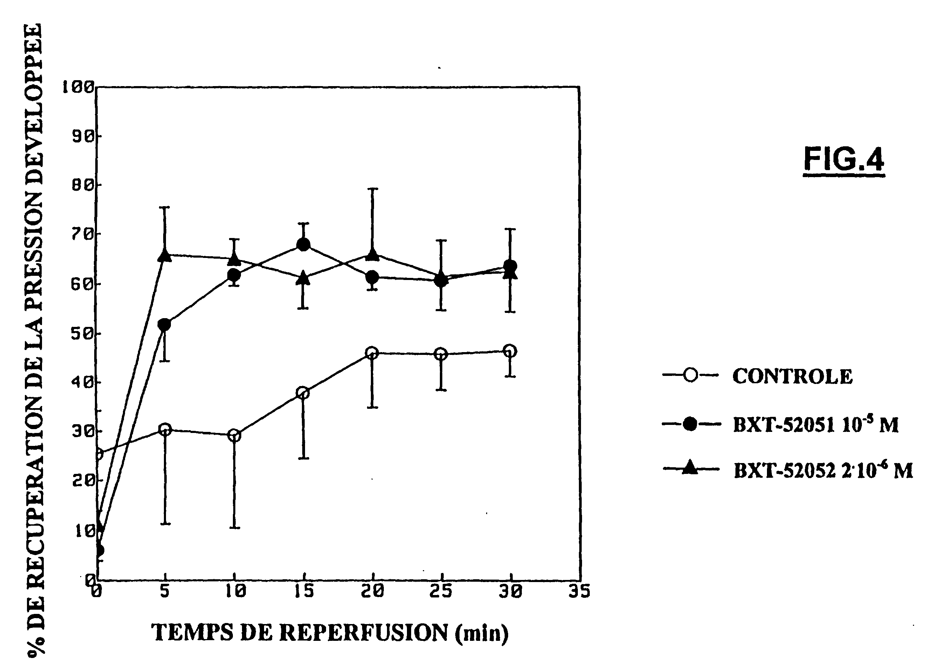

- la figure 4 représente représente les effets des composés de l'invention références BXT 52051 et BXT 52052 sur le pourcentage de récupération de la pression développée lors de la reperfusion d'un coeur de rat isolé soumis à une période d'ischémie, avec en abscisse le temps de reperfusion exprimé en minute et en ordonnée le pourcentage de récupération de la pression développée.

### PARTIE EXPERIMENTALE :

Toutes les réactions ont été effectuées sous atmosphère inerte d'azote, sauf exception notifiée.

Les spectres de masse ont été enregistrés sur un appareil Nermag R10-10B. Le mode d'ionisation utilisé est soit l'impact électronique (EI) à 70 électrons-volts, soit l'ionisation chimique (IC) dans l'ammoniac, soit le bombardement par atomes rapides (FAB) sur une matrice glycérol.

Les spectres ¹H et ¹³C RMN ont été enregistrés sur un appareil Varian Gemini-200. Les déplacements chimiques sont exprimés en ppm par rapport au tétraméthylsilane. Les multiplicités sont exprimées comme suit: "s" pour singulet, "sl" pour singulet large, "d" pour doublet, "t" pour triplet, "q" pour quadruplet et "m" pour multiplet.

Les points de fusion (pF°C) ont été enregistrés sur un appareil Gallenkamp et sont donnés non corrigés.
Le pouvoir rotatoire (α_{D}) a été mesuré sur un appareil Perkin Elmer 241 à 25°C sur la raie D du sodium.

Les purifications par chromatographie liquide sur colonne ont été effectuées, selon les cas, avec de la silice Merck^{**R**} Si60 F₂₅₄ ou avec de la cellulose microcristalline Merck^{**R**}.

### I/ Exemples de synthèse de composés répondant à la formule générale I:

### Exemple 1: Préparation du 3-(2'-mercapto-imidazol-4'-yl)-propanoate d'éthyle: BXT 52021

### A/ Préparation de l'ester éthylique de l'acide urocanique:

L'acide urocanique (Aldrich; 5,52g; 40 mmoles) est suspendu dans 100ml d'éthanol absolu. Le monohydrate de l'acide paratoluènesulfonique (Janssen; 8,36g; 44mmoles) est additionné. Le mélange est chauffé au reflux pendant 12 h. Le solvant est évaporé sous pression réduite. Le résidu est repris par 50 ml d'une solution aqueuse saturée de NaHCO₃ et 75ml d'acétate d'éthyle. La phase organique est séparée, lavée par le même volume d'une solution aqueuse saturée de NaCl (2X50ml). Après séchage de la phase organique sur MgSO₄ et filtration, le solvant est évaporé sous pression réduite. Le résidu ainsi obtenu sous forme d'un solide blanc, est pur (85%).

Caractéristiques physiques :
* point de Fusion : 79°C
* RMN ¹H ( 200MHz, CDCI₃) :
1,30 ppm (t; 3H; J=7,16Hz); 4,22 ppm (q; 2H; J=7,16Hz); 6,41 ppm (d; 1H; J=15,80Hz); 7,31 ppm (s, 1H); 7,60 ppm (d, 1H; J=15,80Hz); 7,72 ppm (s; 1H); 10,16 ppm (sl; 1H).

### B/ Préparation du 3-(imidazol-4'-yl)-propanoate d'éthyle:

Le composé précédent (1,40g; 8,4mmoles) dissous dans 25ml d'éthanol absolu est hydrogéné sous pression (2b) en présence de palladium 10% sur charbon (Aldrich; 100mg) pendant 2h à température ambiante. La suspension est ensuite filtrée sur frité; le solvant est évaporé sous pression réduite. Le composé ainsi obtenu, sous forme d'huile, est utilisé tel quel pour l'étape suivante (rendement: 99%).

Caractéristiques physiques :
* RMN ¹H ( 200MHz, CDCI₃) :
1,21 ppm (t; 2H; J=7,16Hz); 2,64 ppm (t; 2H; J=7,32Hz); 2,92 ppm (t; 2H; J=7,32Hz); 4,10 ppm (q; 2H; J=7,16Hz); 6,78 ppm ( d; 1H; J=1,06Hz); 7,53 ppm (d; 1H; J=1,06Hz); 10,49 ppm (sl; 1H).
* RMN ¹³C ( 50MHz, CDCI₃) :
14,35 ppm (q); 22,27 ppm (t); 34,37 ppm (t); 60,88 ppm (t); 117,99 ppm (d); 135,27 ppm ( d); 136,06 ppm (s); 174,10 ppm (s).

### C/ Préparation du 3-(2'-mercapto-imidazol-4'-yl)-propanoate d'éthyle: BXT 52021

Le 3-(imidazol-4'-yl)-propanoate d'éthyle (1,30g; 7,7mmoles) est mélangé avec du bicarbonate de sodium (3,90g; 46,4mmoles) dans 20ml d'eau déionisée et 20ml d'éther éthylique à température ambiante. Sous une agitation vigoureuse, le chlorothionoformiate de phényle (Lancaster; 2,70ml; 19,5mmoles) est additionné. Le mélange réactionnel est agité pendant 5h à température ambiante. La phase organique est décantée, puis lavée avec une solution saturée de NaCl (2X20ml) et le solvant est évaporé sous pression réduite. Le résidu est repris dans 20ml de méthanol et la solution ainsi obtenue est traitée par de la triéthylamine (Janssen; 3,35ml; 24,0mmoles) pendant 16h à température ambiante. Après évaporation du solvant, le produit désiré est obtenu après purification par chromatographie sur colonne de silice (éluant: AcOEt/Cyclohexane 3/2). (rendement: 75%)

Caractéristiques physiques :
* point de Fusion : 152-154°C (CH₃CO₂Et)
* RMN ¹H ( 200MHz, DMSO-d₆) :
1,15 ppm (t, 3H, J = 7,26Hz) ; 2,58 ppm (m, 4H) ; 4,04 ppm (q, 2H, J = 7,26Hz) ; 6,53 ppm (s, 1H) ; 11,67 ppm (s, 1H) ; 11,88 ppm (s, 1H).
* RMN ¹³C ( 50MHz, DMSO-d₆) :
14,05 ppm (q) ; 19,92 ppm (t) ; 32,16 ppm (t) ; 60,28 ppm (t); 111,70 ppm (d) ; 128,21 ppm (s) ; 160,67 ppm (s) ; 172,18 ppm (s).

### Exemple 2: Préparation de l'acide 3-(2'-mercapto-imidazol-4'-yl)-propanoique: BXT 52020

Le 3-(2'-mercapto-imidazol-4'-yl)-propanoate d'éthyle (200mg; 1mmole), dissous dans 10ml d'un mélange THF/eau (1/1), est saponifié par de la lithine (Aldrich; 84mg; 2mmoles) à température ambiante pendant 16h. Après neutralisation du milieu réactionnel et évaporation à sec, le produit désiré est obtenu après recristallisation dans l'éthanol absolu. (rendement: 93%).

Caractéristiques physiques:
* point de Fusion : 204,5-206,5°C (H₂O)
* RMN ¹H (200MHz, CD₃OD) :
2,57 ppm (m, 2H) ; 2,73 ppm (m, 2H) ; 6,56 ppm (s, 1H).
* RMN ¹³C ( 50MHz, CD₃OD) :
21,23 ppm (t) ; 33,67 ppm (t) ; 113,51 ppm (d) ; 131,14 ppm (s) ; 160,31 ppm (s) ; 176,24 ppm (t).

### Exemple 3 : Préparation du 3-(2'-mercapto-imidazol-4'-yl)-propanamide : BXT 52029

Le 3-(2'-mercapto-imidazol-4'-yl)-propanoate d'éthyle (320 mg, 145 mmoles) est traité avec 10 ml d'une solution aqueuse d'ammoniaque (20 %) pendant 20 h. à température ambiante. Le solvant est évaporé sous pression réduite. Le produit désiré est obtenu après purification par chromatographie liquide sur colonne de silice (éluant: acétate d'éthyle/éthanol 3/1) (161 mg, 58 %).

Caractéristiques physiques :
* point de Fusion : 213-215°C ( CH₃CH₂OH)
* RMN ¹H ( 200MHz, DMSO-d₆) :
2,29 ppm (t, 2H, J = 8,26Hz) ; 2,50 ppm (t, 2H, J = 8,26Hz ); 6,83 ppm (s, 1H) ; 7,31 ppm (s, 1H) ; 11,63 ppm (s, 1H) ; 11,81 ppm (s, 1H).

### Exemple 4 : Préparation de la 2-(2'-mercapto-imidazol-4'-yl)-éthylamine : BXT 52022

### A/Préparation de la 2-(2'-mercapto-imidazol-4'-yl)-Nα-carboxyéthyléthylamine :

La 2-(imidazol-4'-yl)-Na-carboxyéthyléthylamine, obtenue selon la procédure usuelle, est traitée de la même façon que dans l'étape C de l'exemple 1. Le produit ainsi obtenu est utilisé tel quel pour l'étape suivante.

### B/Préparation de la 2-(2'-mercapto-imidazol-4'-yl)-éthylamine : BXT 52022

Le composé obtenu précédemment est hydrolysé par chauffage, au reflux, dans une solution d'acide chlorhydrique concentrée pendant 12h. Le produit désiré est obtenu après recristallisation dans du méthanol.

Caractéristiques physiques :
* point de Fusion : 249-251°C (H₂O)
* RMN ¹H ( 200MHz, D₂O)
2,85 ppm (t, 2H, J = 6,96Hz) ; 3,17 ppm (t, 2H, J = 6,96Hz) ; 6,77 ppm (s, 1H).
* SM ( EI, 70eV) : 143 (M+, 23%) ; 114 (24%) ; 36 (100%).

### Exemple 5 : Préparation de la 2-(2'-mercapto-imidazol-4'-yl)-N,N-diméthyl-éthylamine : BXT 52026

### Préparation de la 2-(imidazol-4'-yl)-N,N-diméthyléthylamine:

A une solution de dichlorhydrate d'histamine (Aldrich, 9,1 g ; 50 mmoles) dans 150 ml d'eau déionisée est ajoutée une solution aqueuse de formaldéhyde (37 % ; 111 ml) et du palladium à 10 % sur charbon (0,29 g). Le mélange est agité vigoureusement sous pression d'hydrogène (5 bars) pendant 6 h. Le catalyseur est filtré puis rincé à l'eau déionisée. Le solvant est évaporé sous pression réduite. Le produit désiré est obtenu sous forme d'une huile (12,7 g) qui est utilisée tel quelle pour la prochaine étape.

Caractéristiques physiques :
* RMN ¹H ( 200MHz, D₂O)
3,18 ppm (t, 2H, J = 7,50Hz) ; 3,44 ppm (t, 2H, J = 7,50Hz) ; 7,33 ppm (s, 1H) ; 8,60 ppm (s, 1H).

### Préparation de la 2-(2'-mercapto-imidazol-4'-yl)-N,N-diméthyléthylamine : BXT 52026

Le produit précedent, traité selon la même procédure que celle décrite pour l'étape C de l'exemple 1, conduit au composé désiré (34%).

Caractéristiques physiques :
* RMN ¹H ( 200MHz, D₂O)
2,28 ppm (s, 6H) ; 2,68 ppm (m, 4H) ; 6,64 ppm (s, 1H).

### Exemple 6 : Préparation de la 2'-mercapto-Nα,Nα-diméthyl-L-(+)-histidine : BXT 52040

L'ester méthylique de la 2'-mercapto-Na,Na-diméthyl-L-(+)-histidine est préparé selon la méthode de préparation suivante:

### A - Préparation du dichlorhydrate de l'ester méthylique de L-(+)-Nα,Nα-diméthylhistidine :

A une solution du dichlorhydrate de l'ester méthylique de la L-(+)-histidine (Janssen; 18,16 g ; 75 mmoles) dans 150 ml d'eau déionisée est ajoutée une solution aqueuse de formaldéhyde 37 % (Janssen ; 12,29 g ; 150 mmoles). Le mélange est hydrogéné sous pression (7b) en présence de Palladium sur charbon 10 % (Aldrich; 1,0 g) pendant 5 h à température ambiante. Le catalyseur est filtré puis rincé à l'eau ; le filtrat est évaporé à sec sous vide pour donner le produit attendu sous forme d'une huile (20,3 g) qui est utilisée directement dans l'étape suivante.

Caractéristiques physiques :
* RMN ¹H (200MHz, D₂O) :
2,91 ppm (s ; 6H) ; 3,50 ppm (m ; 2H) ; 3,72 ppm (s ; 3H) ; 4,48 ppm (dd ; J=5,54-9,04Hz ; 1H) ; 7,38 ppm (s ; 1H) ; 8,61 ppm (s ; 1H).

### B - Préparation de l'ester méthylique de la L-(+)-2'-mercapto-N_{α} ,N_{α}-diméthylhistidine :

Le dichlorhydrate de l'ester méthylique de la L-(+)-N_{α},N_{α} -diméthylhistidine (60,0 g ; 222 mmoles) est dissous dans 750 ml d'eau déionisée. Du bicarbonate de sodium solide ( Labosi ; 130,5 g ; 1,55 moles) est ajouté lentement, suivi de 750 ml de THF. Sous agitation vigoureuse et à une température comprise entre 5-10°C, le chlorothionoformiate de phényle (Lancaster ; 76ml ; 555 mmoles) est additionné pendant 30 min. Le mélange réactionnel est agité, à température ambiante, pendant 260 h. La phase aqueuse est décantée puis extraite avec du chlorure de méthylène (3X150 ml). Les phases organiques sont rassemblées puis évaporées à sec sous vide, à température ambiante. Le résidu est, ensuite, purifié par chromatographie sur colonne de silice en utilisant un gradient d'élution (AcOEt-AcOEt/MeOH=100 %-95/5) pour donner 24,0 g d'un produit pur. Ce produit (5,0 g) est suspendu dans 150 ml de chlorure de méthylène puis filtré. Le résidu obtenu après évaporation du solvant du filtrat conduit à 4,42 g d'un produit énantiomériquement pur (Rendement = 42 %).

La pureté énantiomérique est déterminée par ¹H RMN dans CDCl₃ avec 3 mg d'échantillon et 10 mg de Eu(tfc)₃.

Caractéristiques physiques :
* pF : 170-171°C
* RMN ¹H (200MHz, CDCI₃) :
2,39 ppm (s ; 6H) ; 2,78 ppm (d ; J=7,3Hz ; 2H) ; 3,38 ppm (t ; J=7,30Hz ; 1H) ; 3,72 ppm (s ; 3H) ; 6,44 ppm (s ; 1H) ; 10,08 ppm (s1 ; 1H) ; 10,24 (s1 ; 1H).
* RMN ¹³C (50MHz, DMSO) : 24,52 ppm (t) ; 41,03 ppm (q) ; 51,03 ppm (d) ; 65,04 ppm (q) ; 112,74 ppm (d) ; 126,13 ppm (s) ; 160,35 ppm (s) ; 171,17 ppm (s).
* SM (EI, 70Ev) :
229 (M+, 25), 170 (8), 116 (100).
* α_{D} (c = 1,0 ; MeOH) = +31,2°

L'ester méthylique de la 2'-mercapto-Nα,Nα-diméthyl-L-(+)-histidine ainsi obtenu est alors saponifié à l'aide d'hydroxyde de lithium selon la procédure habituelle. (rendement: 80%)

Caractéristiques physiques :
* point de Fusion ; 274°C (dec.)
* RMN ¹H ( 200MHz, D₂O) :
2,83 ppm (s; 6H); 3,03 ppm (dd; 1H; J=8,14-15,60Hz); 3,17 ppm (dd; 1H; J=5,54-15,60Hz); 3,75 ppm (dd; 1H; J=5,54-8,14Hz); 6,76 ppm (s, 1H).

### Exemple 7: Préparation de la N-2-[3-(2'-mercaptoimidazol-4'-yl)-propanoyloxy]-éthyl-N'-méthylpipérazine: BXT 52055

### A/ Préparation de la N-(2-hydroxyéthyl)-N'-méthylpipérazine:

La N-méthylpipérazine (10g; 100mmoles) et le 2-chloroéthanol (8,05g; 100mmoles) sont agités à 100°C pendant 4h. Le milieu réactionnel, très visqueux, est additionné de 250ml d'acétone et la suspension résultante est neutralisée par 15ml de triéthylamine. Après filtration du chlorhydrate de triéthylamine, le solvant est évaporé sous pression réduite. Le composé désiré est obtenu après purification par chromatographie sur colonne d'alumine ( Merck ^{**R**}; Aluminium Oxide 90; 63-200µm; éluant: Acétate d'éthyle) sous la forme d'une huile incolore. Rendement: 75%

Caractéristiques physiques:
* RMN ¹H (200MHz, CDCI₃) :
2,20 ppm (s; 3H); 2,39 ppm (m; 8H); 2,46 ppm (t; 2H; J= 5,5Hz); 3,41 ppm (sl; 1H); 3,54 ppm (t; 2H; J= 5,5Hz).

### B/ Préparation de la N-2-[3-(2'-mercaptoimidazol-4'-yl)-propanoyloxy]-éthyl-N'-méthyl-pipérazine: BXT 52055

Le dérivé 3-(2'-mercaptoimidazol-4'-yl)-propanoate de méthyle, préparé selon la procédure décrite dans l'exemple **1**, ( 0,35g; 1,88mmoles) finement broyé est placé dans de la N-(2-hydroxyéthyl)-N'-méthylpipérazine ( 5,0g; 35mmoles) en présence de cyanure de potassium (0,12g; 1,88mmoles). Le milieu réactionnel est agité à 100°C pendant 15h. Après distillation de la N-(2-hydroxyéthyl)-N'-méthylpipérazine, en excès, sous pression réduite ( p= 0,5mm de Hg; T°= 100°C), le résidu est chromatographié sur colonne d'alumine ( Merck ^{**R**}; Aluminium Oxide 90; 63-200µm; éluant: Acétate d'éthyle/Méthanol 6/1). Le composé désiré est obtenu, après recristallisation dans l'acétate d'éthyle, avec un rendement de 80%.

Caractéristiques physiques:
* pF : 172-174°C (acétate d'éthyle)
* RMN ¹H (200MHz, CD₃COCD₃ + D₂O) :
2,15 ppm (s; 3H); 2,43 ppm (m; 8H); 2,58 ppm (t; 2H; J= 5,6Hz); 2,69 ppm (m; 4H); 4,16 ppm (t; 2H; J= 5,6Hz); 6,61 ppm (s; 1H).
* RMN ¹³C (50MHz, D₂O) :
22,29 ppm; 35,20 ppm; 46,88 ppm; 54,38 ppm; 55,83 ppm; 58,01 ppm; 64,66 ppm; 115,65 ppm; 115,88 ppm; 132,57 ppm; 157,81 ppm; 177,66 ppm.
* SM (IC; NH₃) :
299 (MH⁺; 100%); 281 (20%); 267 (85%); 250 (34%); 233 (20%); 187 (15%); 172 (18%); 145 (18%); 127 (30%).

### Exemple 8: Préparation de l'acide 2-[3-(2'-mercaptoimidazol-4'-yl)-propanamidol-éthanesulfonique : BXT 52053

A une solution de l'acide 3-(2'-mercaptoimidazol-4'-yl)-propanoique BXT 52020 (0,69g; 4mmoles), et de N-hydroxysuccinimide (0,46g; 4mmoles) dans 30ml de THF anhydre, à 0-5°C et sous agitation, est additionnée goutte à goutte une solution de N,N-dicyclohexylcarbodiimide (0,84g; 4mmoles) dans 10ml de THF. L'agitation est maintenue pendant 16h à cette température. Après évaporation du solvant sous pression réduite, le résidu solide est repris dans 30ml d'acétone anhydre. La N,N'-dicyclohexylurée est éliminée par filtration et le filtrat, refroidi à 10°C, est additionné d'une solution de taurine (0,5g; 4mmoles) dans 10ml d'eau déionisée, contenant du bicarbonate de sodium (0,34g; 4mmoles). Le mélange réactionnel est agité pendant 1h à température ambiante. Après évaporation du solvant sous pression réduite, le composé désiré est obtenu, après purification par chromatographie sur colonne de silice greffée ( Merck ^{**R**}; RP-8; 40-63µm) avec un éluant eau-méthanol 9/1. Il est recristallisé dans un mélange méthanol/éthanol. ( rendement: 60%).

Caractéristiques physiques:
* pF : 210°C (dec.)
* RMN ¹H (200MHz, D₂O) :
2,47 ppm (t; 2H; J= 7,1Hz); 2,74 ppm (t; 2H; J= 7,1Hz); 2,96 ppm (t; 2H; J= 6,8Hz); 3,47 ppm (t; 2H; J= 6,8Hz); 6,62 ppm (s; 1H).
* RMN ¹³C (50MHz, D₂O) :
23,09 ppm; 37,02 ppm; 37,67 ppm; 52,35 ppm; 115,91 ppm; 116,03 ppm; 132,47 ppm; 157,66 ppm; 177,59 ppm.
* SM (FAB négatif; glycérol) :
278 (M-; 100%); 255 (10%); 183 (64%); 124 (14%).

### Exemple 9: Préparation du chlorure de 3-(2'-mercaptoimidazol-4'-yl)-propanoate de choline: BXT 52054

### A/ Préparation du 3-(2'-mercaptoimidazol-4'-yl)-propanoate de 2-(N,N-diméthylamino)-éthyle:

Une solution du 3-(2'-mercaptoimidazol-4'-yl)-propanoate d'éthyle (voir l'exemple **1**) (1,40g; 7mmoles) dans 20ml de 2-(N,N-diméthylamino)-éthanol est chauffée en présence de cyanure de potassium (0,75g; 3,9mmoles) à 80°C pendant 4h. Après évaporation du 2-(N,N-diméthylamino)-éthanol sous pression réduite, le produit désiré est obtenu et utilisé tel quel pour l'étape suivante.

### B/ Préparation du 3-(1'-tert-butoxycarbonyl-2'-tert-butoxycarbonylthioimidazol-4'-yl)-propanoate de 2-(N,N-diméthylamino)-éthyle:

A une solution du produit précédent dans 50ml de chlorure de méthylène sont additionnés le pyrocarbonate de di-*tert*-butyle (3,3g; 15mmoles) et de la triéthylamine (5ml; 36mmoles) ainsi que de la 4-diméthylaminopyridine (lOmg). Le mélange réactionnel est agité à température ambiante pendant 2h. Après addition de 30ml d'eau, la phase organique est décantée, séchée sur sulfate de magnésium et filtrée. Le solvant est évaporé sous pression réduite. Le résidu ainsi obtenu est purifié par chromatographie sur colonne de silice ( éluant: acétone) pour conduire au produit désiré, obtenu sous d'une huile incolore.
Rendement global pour ces 2 étapes: 75%

Caractéristiques physiques:
* RMN ¹H (200MHz, CDCI₃) :
1,46 ppm (s; 9H); 1,57 ppm (s; 9H); 2,25 ppm (s; 6H); 2,53 ppm (t; 2H; J= 5,86Hz); 2,69 ppm (m; 2H); 2,86 ppm (m; 2H); 4,16 ppm (t; 2H; J= 5,86Hz); 7,33 ppm (s, 1H).
* RMN ¹³C (50MHz, CDCI₃) :
28,00 ppm; 28,30 ppm; 33,30 ppm; 45,98 ppm; 58,04 ppm; 62,62 ppm; 86,18 ppm; 87,12 ppm; 119,15 ppm; 135,74 ppm; 142,17 ppm; 147,14 ppm; 165,80 ppm; 173,45 ppm.
* SM (IC; NH₃) :
444 (MH⁺; 100%); 344 (18%).

### C/ Préparation de l'iodure de 3-(1'-tert-butoxycarbonyl-2'-tert-butoxycarbonylthio-imidazol-4'-yl)-propanoate de choline:

Une solution du 3-(1'-*tert*-butoxycarbonyl-2'-tert-butoxycarbonylthioimidazol-4'-yl)-propanoate de 2-(N,N-diméthylamino)-éthyle (0,81g; 1,8mmoles) dans 20ml de THF est traitée avec de l'iodure de méthyle (0,6ml; 9,6mmoles) à température ambiante pendant 2h. L'excès d'iodure de méthyle ainsi que le solvant sont évaporés sous pression réduite pour conduire au produit désiré qui est suffisamment pur pour être utilisé tel quel pour l'étape suivante.
Rendement: 100%

Caractéristiques physiques:
* RMN ¹H (200MHz, CDCI₃) :
1,44 ppm (s; 9H); 1,56 ppm (s; 9H); 2,73 ppm (m; 2H); 2,86 ppm (m; 2H); 3,48 ppm (s; 9H); 4,06 ppm (m; 2H); 4,55 ppm (m; 2H); 7,37 ppm (s; 1H).
* RMN ¹³C (50MHz, CDCI₃) :
23,23 ppm; 27,98 ppm; 28,38 ppm; 33,41 ppm; 55,11 ppm; 58,27 ppm; 65,45 ppm; 86,63 ppm; 87,64 ppm; 119,50 ppm; 135,62 ppm; 141,48 ppm; 146,98 ppm; 165,71 ppm; 172,41 ppm.
* SM (FAB positif; glycérol) :
458 (M⁺)

### D/ Préparation du chlorure de 3-(2'-mercaptoimidazol-4'-yl)-propanoate de choline:

Le précédent composé (1,07g; 1,8mmoles) est mis en solution dans 7,5ml de chlorure de méthylène puis traité avec 7,5ml d'acide trifluoroacétique à température ambiante pendant 2h. Après évaporation du solvant et de l'acide trifluoroacétique, en excès,sous pression réduite, le résidu est purifié par chromatographie sur colonne de silice ( éluant: Acétate d'éthyle-Méthanol 2/1) pour conduire au produit désiré.
Rendement: 77%

Caractéristiques physiques:
* RMN ¹H (200MHz, D₂O) :
2,75 ppm (m; 4H); 3,11 ppm (s; 9H); 3,64 ppm (m; 2H); 4,48 ppm (m; 2H); 6,65 ppm (s; 1H).
* RMN ¹³C (50MHz, D₂O) :
22,16 ppm; 35,07 ppm; 56,28 ppm; 61,00 ppm; 67,42 ppm; 115,90 ppm; 132,41 ppm; 157,96 ppm; 176,53 ppm.
* SM (FAB positif; glycérol) :
258 (M⁺)

### Exemple 10: Préparation du 3-(2'-mercaptoimidazol-4'-yl)-propanoate de carnitine: BXT 52052

A une solution de l'acide 3-(2'-mercaptoimidazol-4'-yl)-propanoique (voir l'exemple **2**) (0,86g; 5mmoles) et de N-hydroxysuccinimide (0,60g; 5,2mmoles) dans 80ml de THF est additionné le dicyclohexylcarbodiimide (1,1g; 5,3mmoles). Le mélange réactionnel est agité à température ambiante pendant 5h. Le solvant est évaporé sous pression réduite pour conduire au mélange de l'ester activé désiré et de la N,N'-dicyclohexylurée, qui sera utilisé tel quel pour l'étape suivante. L'ester *p*-méthoxybenzylique de carnitine, préparé à partir de carnitine (0,81g; 5mmoles) et de chlorure de *p*-méthoxybenzyle (0,79g; 5mmoles) dans 30ml de DMF anhydre à 80°C pendant 5h, est mélangé avec l'ester activé de l'acide 3-(2'-mercaptoimidazol-4'-yl)propanoique préparé précédemment. Le mélange réactionnel est ensuite traité avec de la diisopropyléthylamine (1,0ml; 5,8mmoles) à température ambiante pendant 16h. La N,N'-dicyclohexylurée est filtrée, et le solvant est évaporé sous pression réduite. Le résidu ainsi obtenu est traité avec 7,5ml de l'acide 3-mercaptopropionique et 7,5ml d'acide trifluoroacétique à température ambiante pendant 2h. L'acide trifluoroacétique est évaporé sous pression réduite. Le résidu est repris avec 30ml d'eau. L'excès d'acide 3-mercaptopropionique est extrait avec de l'éther éthylique (3x20ml). La solution aqueuse est neutralisée à l'aide de bicarbonate de sodium puis lyophilisée. Le composé désiré est obtenu, après purification par chromatographie sur colonne de silice greffée ( Merck ^{**R**}; Diol; 40-63µm) avec un éluant acétate d'éthyle-méthanol 1/3.
Rendement global: 30%

Caractéristiques physiques:
* pF : 81°C
* RMN ¹H (200MHz, D₂O) :
2,39 ppm (dd; 1H; J= 7,44-15,53Hz); 2,53 pm (dd; 1H; J= 5,67-15,53Hz); 2,75 ppm (m; 4H); 3,06 ppm (s; 9H); 3,53 ppm (d; 1H; J= 14,38Hz); 3,75 ppm (dd; 1H; J= 8,46-14,38Hz); 5,55 ppm (m; 1H); 6,66 ppm (s; 1H).
* RMN ¹³C (50MHz, D₂O) :
22,19 ppm; 35,54 ppm; 42,74 ppm; 56,30 ppm; 69,82 ppm; 70,81 ppm; 116,20 ppm; 132,53 ppm; 160,65 ppm; 176,22 ppm; 179,33 ppm.
* SM (FAB positif; glycérol) :
316 (MH⁺)
* [α]_{D} : -36,1° (c=1,0; H₂O).

### Exemple 11: Préparation du chlorure de l'ester 2-(triméthylammonium)-éthylique de la 2'-mercapto-histidine : BXT 52058

### A/ Préparation de l'ester méthylique de la Nα,Nτ-bis-terbutyloxycarbonyl-L-histidine:

A un mélange du dichlorhydrate de l'ester méthylique de la L-histidine (4,84g; 20mmoles) dans un mélange eau-THF (20/40) est ajouté le carbonate de sodium (9,33g; 88mmoles). En agitant vigoureusement le mélange obtenu, est additonné le pyrocarbonate de di-terbutyle (10g; 46mmoles). Après 1,5h d'agitation à température ambiante, le milieu réactionnel est décanté. La phase aqueuse est extraite avec 50ml d'acétate d'éthyle. Les phases organiques de décantation et d'extraction sont rassemblées, le solvant est évaporé sous pression réduite. Le produit brut est purifié par chromatographie liquide sur colonne de silice (éluant: Acétate d'éthyle-cyclohexane 1/1)
Rendement: 78%

Caractéristiques physiques:
* RMN ¹H (200MHz, CDCI₃) :
1,40 ppm (s; 9H); 1,57 ppm (s; 9H); 3,02 ppm (d; 2H; J=5,34Hz); 3,70 ppm (s; 3H); 4,54 ppm (m; 1H); 5,68 ppm (d; 1H; J=8,06Hz); 7,11 ppm (d; 1H; J=1,02Hz); 7,96 ppm (d; 1H, J=1,02Hz).
* RMN ¹³C (50MHz, CDCI₃) :
28,03 ppm; 28,47 ppm; 30,39 ppm; 52,54 ppm; 53,39 ppm; 80,04 ppm; 85,94 ppm; 114,98 ppm; 137,40 ppm; 139,09 ppm; 147,35 ppm; 156,00 ppm; 172,89 ppm.

### B/ Préparation de l'ester méthylique de la 2'-mercapto-Nα-terbutyloxycarbonyl-L-histidine:

Le produit désiré est obtenu selon une procédure similaire à celle décrite dans la partie C de l'exemple 1.
Rendement: 32%

Caractéristiques physiques:
* pF : 73-75°C
* RMN ¹H (200MHz, CDCI₃) :
1,39 ppm (s; 9H); 2,96 ppm (dd; 1H; J=7,60-15,40Hz); 3,04 ppm (dd; 1H; J=7,60-15,40Hz); 3,74 ppm (s; 3H); 4,57 ppm (m; 1H); 5,55 ppm (d; 1H; J=6,76Hz); 6,58 ppm (s; 1H); 11,46 ppm (s; 1H); 11,53 ppm (s; 1H).
* RMN ¹³C (50MHz, CDCI₃) :
26,26 ppm; 28,08 ppm; 51,90 ppm 52,78 ppm; 78,67 ppm; 110,58 ppm; 125,12 ppm; 155,73 ppm; 160,56 ppm; 172,50 ppm.

### C/ Préparation de l'ester 2-(N,N-diméthylamino)-éthylique de l'acide 3-(1'-terbutyloxycarbonyl-2'-terbutyloxycarbonylthio-imidazol-4'-yl)-2-terbutyloxycarbonylamino-propanoïque:

A une solution du précédent produit (760mg; 1,9mmoles) dans 5ml de 2-(N,N-diméthylamino)-éthanol est additionné du cyanure de potassium (0,25g; 1,3mmoles). Le mélange réactionnel est chauffé à 80°C pendant 2h. L'excès de 2-(N,N-diméthylamino)-éthanol est distillé. Le résidu de distillation est repris avec 10ml de chlorure de méthylène. Le pyrocarbonate de di-terbutyle (1,09g; 5mmoles) et la triéthylamine (0,83ml; 6mmoles) sont ajoutés à cette solution. Le mélange ainsi obtenu est traité avec de la 4-diméthylaminopyridine (5mg) à température ambiante pendant 1h. Le solvant est évaporé sous pression réduite. Le produit brut est purifié par chromatographie liquide sur colonne de silice (éluant: Acétone)
Rendement: 38%

Caractéristiques physiques:
* RMN ¹H (200MHz, CDCI₃) :
1,35 ppm (s; 9H); 1,41 ppm (s; 9H); 1,52 ppm (s; 9H); 2,18 ppm (s; 6H); 2,48 ppm (t; 2H; J=5,78Hz); 2,99 ppm (d; 2H; J=5,12Hz); 4,15 ppm (m; 2H); 4,50 ppm (m; 1H); 5,54 ppm (d; 1H; J=8,06Hz); 7,34 ppm (s; 1H).
* RMN ¹³C (50MHz, CDCI₃) :
27,93 ppm; 28,24 ppm; 28,49 ppm; 30,60 ppm; 45,87 ppm; 53,33 ppm; 57,77 ppm; 63,35 ppm; 80,03 ppm; 86,38 ppm; 87,15 ppm; 120,44 ppm; 136,00 ppm; 138,61 ppm; 146,96 ppm; 155,94 ppm; 165,54 ppm; 172,26 ppm.

### D/ Préparation du chlorure de l'ester 2-(triméthylammonium)-éthylique de la 2'-mercapto-histidine : BXT 52058

Une solution du produit précédent (560mg; 1,0mmoles) dans 8ml de THF est traitée avec de l'iodure de méthyle (0,10ml; 1,6mmoles) à température ambiante pendant 2h. L'excès d'iodure de méthyle ainsi que le solvant sont évaporés sous pression réduite. Une solution du résidu ainsi obtenu dans 10ml de chlorure de méthylène est percolée avec de l'acide chlorhydrique gazeux pendant 1h à température ambiante. Le solvant est évaporé sous pression réduite. Le produit obtenu est recristallisé dans un mélange éther éthylique-éthanol.
Rendement: 80%

Caractéristiques physiques:
* pF : 141°C (décomposé)
* RMN ¹H (200MHz, D₂O) :
3,10 ppm (s; 9H); 3,16 ppm (d; 2H; J=7,50Hz); 3,65 ppm (m; 2H); 4,40 ppm (t; 1H; J=7,50Hz); 4,65 ppm (m; 2H); 6,85 ppm (s; 1H).

### II/ ESSAIS DEMONTRANT L'ACTIVITE THERAPEUTIQUE/ PHARMACOLOGIQUE:

Les protocoles opératoires décrits ci-après permettent de démontrer l'activité thérapeutique/pharmacologique revendiquée des composés de l'invention de formule (I) précitée.

### Exemple 12 : PIEGEAGE DE LA FERRYLMYOGLOBINE :

La capacité des molécules de la présente invention à piéger la ferrylmyoglobine, est mise en évidence par une mesure cinétique de la disparition de la ferrylmyoglobine à 590 nm, à pH 7,3 et à 25°C.

Le tampon pH 7,3 est obtenu par titration de phosphate de potassium 50 mM, contenant 0,1 mM d'acide diéthylènetriaminepentaacétique (DTPA).

La ferrylmyoglobine est formée en incubant, dans le tampon défini précédemment, 50 µM de metmyoglobine (purifiée à partir de coeur de cheval) avec 200 µM de peroxyde d'hydrogène (H₂O₂) pendant 4 minutes à 25°C.

L'excès de H₂O₂ est ensuite détruit en ajoutant environ 220 U/ml de catalase dans ce milieu réactionnel.

Cinq minutes après ajout de H₂O₂, on supplémente alors le milieu réactionnel avec une molécule selon la présente invention (à 25 ou 100 pM en concentration finale), et on suit la cinétique de disparition de la ferrylmyoglobine pendant 5 minutes, à une longueur d'onde d'absorbance égale à 590 nm.

Un exemple des cinétiques expérimentales obtenues dans cette étude est présenté sur la figure 1 dans le cas du BXT-52021.

L'effet de la molécule testée est mesuré en déterminant le pourcentage de ferrylmyoglobine piégée au temps 2 minutes, la valeur de 100 % étant déterminée en l'absence de la molécule, dans les mêmes conditions expérimentales.

Les résultats obtenus sont présentés dans le tableau 1.

Ces résultats montrent que les molécules décrites dans la présente invention piègent très efficacement la ferrylmyoglobine.

### Exemple 13 : PREVENTION DE L'INACTIVATION DE LA GLUTATHION PEROXYDASE PAR L'ACIDE HYPOCHLOREUX :

Dans un tampon phosphate de potassium 50 mM pH 7,0 contenant 0,1 mM de DTPA, une quantité de glutathion peroxydase érythrocytaire d'origine bovine est mise à incuber, pendant 2 minutes à 37°C, à la concentration finale de 1,5 U/ml, en présence de 10 µM d'acide hypochloreux, en présence ou non d'une molécule décrite dans la présente invention (à 25 µM).

Au bout de 2 minutes d'incubation, on ajoute alors 100 µl de ce milieu réactionnel dans 1,5 ml de tampon Tris 50 mM pH 7,6 (à 37°C) contenant 0,1 mM de DTPA, 0,165 mM de β-NADPH, 2,2 mM de glutathion réduit et 1,1 U/ml glutathion disulfure réductase.

Puis après ajout de 50 µl d'hydroperoxyde de t-butyle à 6,6 mM, l'activité glutathion peroxydase est dosée à 37°C en mesurant pendant 2 minutes la diminution d'absorbance à 340 nm.

Les résultats obtenus sont présentés dans le tableau 2. Ils sont exprimés en pourcentage de l'activité glutathion peroxydase dosée dans les mêmes conditions expérimentales, en l'absence d'acide hypochloreux et de la molécule testée.

Ces résultats montrent que les molécules décrites dans la présente invention bloquent très efficacement l'inactivation de la glutathion peroxydase par l'acide hypochloreux.

### Exemple 14 : PREVENTION DE L'INACTIVATION DE LA GLUCOSE-6-PHOSPHATE DESHYDROGENASE PAR LE SYSTEME Cu(II)/ ASCORBATE/O₂ :

Dans un tampon phosphate de potassium 50 mM pH 7,0, une quantité de glucose-6-phosphate deshydrogénase (purifiée à partir de Leuconostoc *mesenteroides*) est mise à incuber, pendant 5 minutes à 37°C, à la concentration finale de 1,2 U/ml, en présence de sulfate de cuivre à 4,5 µM et d'ascorbate à 360 µM, en présence ou non d'une molécule décrite dans la présente invention (à 25 µM).

Au bout de 5minutes d'incubation, on ajoute alors 20 µl de ce milieu réactionnel dans 980 µl de tampon Tris 50 mM pH 7,5 (à 37°C) contenant 0,380 mM de β-NADP⁺, 3,3 mM de glucose-6-phosphate et 6,3 mM de chlorure de magnésium.

Puis l'activité glucose-6-phosphate deshydrogénase est dosée à 37°C en mesurant pendant 7 minutes l'augmentation d'absorbance à 340 nm.

Les résultats obtenus sont présentés dans le tableau 3. Ils sont exprimés en pourcentage de l'activité glucose-6-phosphate deshydrogénase dosée dans les mêmes conditions expérimentales, en l'absence du système Cu(II)/ascorbate/O₂ et de la molécule testée.

Ces résultats montrent que les molécules décrites dans la présente invention bloquent très efficacement l'inactivation de la glucose-6-phosphate deshydrogènase par le système Cu(II)/ascorbate/O_{2.}

### Exemple 15 : PREVENTION DE LA DEGRADATION D'ADN PAR LE SYSTEME Fe(II)-CITRATE/H₂O₂/ASCORBATE :

Dans un tampon phosphate de potassium 50 mM pH 7,3, de l'ADN double brin (purifié à partir de phage lambda) à 10 µg/ml est mis à incuber, pendant 1 heure à 37°C, avec 20 µM du complexe Fe^{II}-citrate, 200 µM de H₂O₂ et 200 µM d'ascorbate, en présence ou non d'une molécule décrite dans la présente invention (à 100 µM).

Au bout de cette heure d'incubation, 220 U/ml de catalase sont alors ajoutées au milieu réactionnel et l'intégrité de l'ADN est mesurée par spectrofluorimétrie après ajout dans le milieu réactionnel de 10 µl d'une solution de bromure d'éthidium à 5 mM (longueur d'onde d'excitation : 510 nm ; longueur d'onde d'émission: 590 nm).

L'effet de la molécule testée est mesuré en déterminant le pourcentage d'ADN intact, la valeur prise égale à 100% étant déterminée dans les mêmes conditions expérimentales, en l'absence du système Fe(II)-citrate/H₂O₂/ascorbate et de la molécule testée.

Les résultats obtenus sont présentés dans le tableau 4.

Ces résultats montrent que les molécules de la présente invention préviennent très significativement la dégradation d'ADN par le système Fe(II)-citrate/H₂O₂/ascorbate.

### Exemple 16 : INHIBITION DE LA NECROSE CARDIAQUE INDUITE PAR UNE PERIODE D'ISCHEMIE-REPERFUSION : CAS DU COMPOSE BXT 52021

L'effet cardioprotecteur des molécules décrites dans la présente invention a été mis en évidence dans un modèle expérimental de coeur isolé perfusé.

Une solution d'héparinate de sodium est injectée par voie intrapéritonéale (40 unités/100 g) à des rats mâles Sprague-Dawley de poids compris entre 250 et 350 grammes. Les rats sont anesthésiés à l'éther 30 minutes après. Le coeur est alors prélevé rapidement et il est ensuite perfusé selon la méthode de Langendorff (voir O. LANGENDORFF; Pflügers Arch. Physiol. Mensch. Tiere; (1895); 61; page 291), sous stimulation électrique de fréquence 300/min. La solution de perfusion, équilibrée avec 95 % d'oxygène et 5 % de gaz carbonique, est constituée par un tampon de Krebs-Henseleit (pH 7,4) modifié, dont la composition est la suivante : NaCl 118 mM, KCI 4,7mM, MgSO₄ 1,2 mM, CaCl₂ 1 mM, KH₂PO₄ 1,2 mM, NaHCO₃ 25mM et glucose 11 mM. L'ensemble du montage est thermostaté à 37°C.

L'état de fonctionnement du coeur est suivi par la mesure des paramètres hémodynamiques suivants: fréquence cardiaque, pressions ventriculaires systolique et télédiastolique (dont la différence exprime la pression développée), pentes minimale et maximale des variations de pressions en fonction du temps.

Lorsque ces paramètres hémodynamiques sont stabilisés (soit environ 30 minutes après le démarrage de la perfusion), le coeur est soumis à une période d'ischémie par arrêt de la perfusion et arrêt de la stimulation électrique. La reperfusion est ensuite déclenchée avec remise en route de la stimulation électrique lorsque la pression ventriculaire atteint une valeur maximale (pic de contracture myocardique dû à l'ischémie).

L'étude du BXT-52021 dans ce modèle expérimental est effectuée en perfusant le coeur avec le milieu de perfusion contenant 2 µM de BXT-52021 pendant 12 minutes préalablement à la période d'ischémie, ainsi que lors de la reperfusion.

A l'examen des résultats obtenus en enregistrant les paramètres hémodynamiques cités précédemment, on constate que lorsque le coeur est soumis à une ischémie-reperfusion, sa pression télédiastolique augmente et sa pression systolique diminue, alors que celle-ci est maintenue en présence du BXT-52021.

En outre, l'altération du tissu myocardique est évaluée en déterminant la concentration dans le perfusat de deux enzymes cytosoliques libérées dans le milieu de perfusion, la créatine phosphokinase (CPK) et la lactate deshydrogénase (LDH). Les concentrations de ces deux enzymes sont exprimées en unités d'activité enzymatique par litre de perfusat.

Ainsi, en effectuant la mesure au cours du temps de ces deux paramètres, l'altération du tissu myocardique (dûe à l'ischémie-reperfusion) ou sa protection (effet du BXT-52021) sont mises en évidence respectivement par une augmentation ou une diminution des concentrations en CPK et en LDH dans le perfusat.

Les résultats sont présentés sur la figure 2.

Ces résultats montrent que les molécules selon la présente invention permettent de protéger très efficacement les cellules myocardiques d'une nécrose due à une ischémie ou à une reperfusion post-ischémique.

### Exemple 17: INHIBITION DE LA NECROSE CARDIAQUE INDUITE PAR UNE PERIODE D'ISCHEMIE-REPERFUSION : CAS DU COMPOSE BXT 52053

Dans le même modèle expérimental que celui décrit dans l'exemple 16, on a modifié la composition de la solution de perfusion de telle manière qu'elle contienne CaCl₂ 2,5 mM et MgCl₂ 2,4 mM (toutes choses étant égales par ailleurs). L'effet du BXT-52053 est alors comparé à celui de l'albumine et de la L-ergothionéine.

Cette étude est effectuée en perfusant le coeur avec la solution de perfusion contenant 20 µM de BXT-52053, ou 600 µM d'albumine (BSA), ou 100 µM de L-ergothionéine, pendant les 12 minutes précédant la période d'ischémie, puis pendant les 30 premières minutes de reperfusion post-ischémique

L'altération du tissu myocardique dûe à l'ischémie et/ou à la reperfusion est évaluée en déterminant la quantité totale de créatine phosphokinase (CPK) libérée dans le perfusat pendant la période de reperfusion. Les résultats sont exprimés en milliunités d'activité enzymatique par milligramme de coeur et par minute.

La protection du tissu myocardique est alors mise en évidence par une diminution de la quantité totale de CPK libérée dans le perfusat pendant les 30 minutes de reperfusion.

Les résultats sont présentés sur la figure 3.

Ces résultats montrent que l'albumine ou la L-ergothionéine n'ont pas d'effet significatif alors que les molécules selon la présente invention permettent de protéger très efficacement les myocytes cardiaques et par conséquent de diminuer l'étendue de la nécrose cardiaque dûe à une ischémie et/ou à une reperfusion post-ischémique.

### Exemple 18: AMELIORATION DE LA RECUPERATION DE LA PRESSION DEVELOPPEE D'UN COEUR SOUMIS A UNE PERIODE D'ISCHEMIE-REPERFUSION :

Dans le même modèle expérimental que celui décrit dans l'exemple 17, l'étude du BXT-52051 et du BXT-52052 est effectuée en perfusant le coeur avec le milieu de perfusion contenant 10 µM de BXT-52051 ou 2 µM de BXT-52052 pendant 12 minutes préalablement à la période d'ischémie, ainsi que lors de la reperfusion.

L'altération de la fonction cardiaque est évaluée à partir de la mesure de paramètres hémodynamiques (pressions systolique et télédiastolique ventriculaires) en déterminant au cours du temps le pourcentage de récupération de la pression développée (différence entre pression systolique et télédiastolique) lors de la reperfusion, par rapport à celle enregistrée juste avant la période d'ischémie (pression développée stabilisée).

Les résultats sont présentés sur la figure 4.

A l'examen des résultats obtenus, on constate que lorsque le coeur est soumis à une ischémie-reperfusion, la pression développée ventriculaire remonte lentement jusqu'à une valeur seuil, alors qu'elle est récupérée beaucoup plus rapidement et atteint une valeur supérieure en présence du BXT-52051 ou du BXT-52052.

Ces résultats montrent que les molécules selon la présente invention permettent d'améliorer la récupération de la fonction ventriculaire du coeur lorsque celui-ci est soumis à une ischémie puis reperfusé.

**TABLEAU 1**

| Molécule testée | Concentration (µM) | % de ferrylmyoglobine piégée |
|---|---|---|
| | 25 | 53,1 |
| BXT-52020 | | |
| | 100 | 71,5 |
| | 25 | 52,4 |
| BXT-52021 | | |
| | 100 | 69,5 |
| | 25 | 36,9 |
| BXT-52022 | | |
| | 100 | 61,0 |
| | 25 | 51,1 |
| BXT-52029 | | |
| | 100 | 70,0 |
| | 25 | 41,0 |
| BXT-52030 | | |
| | 100 | 62,3 |

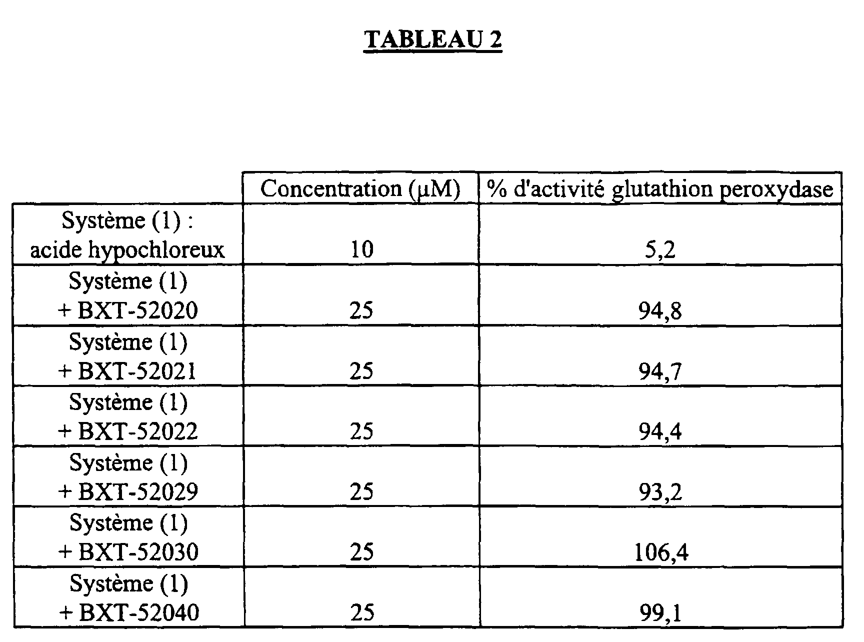

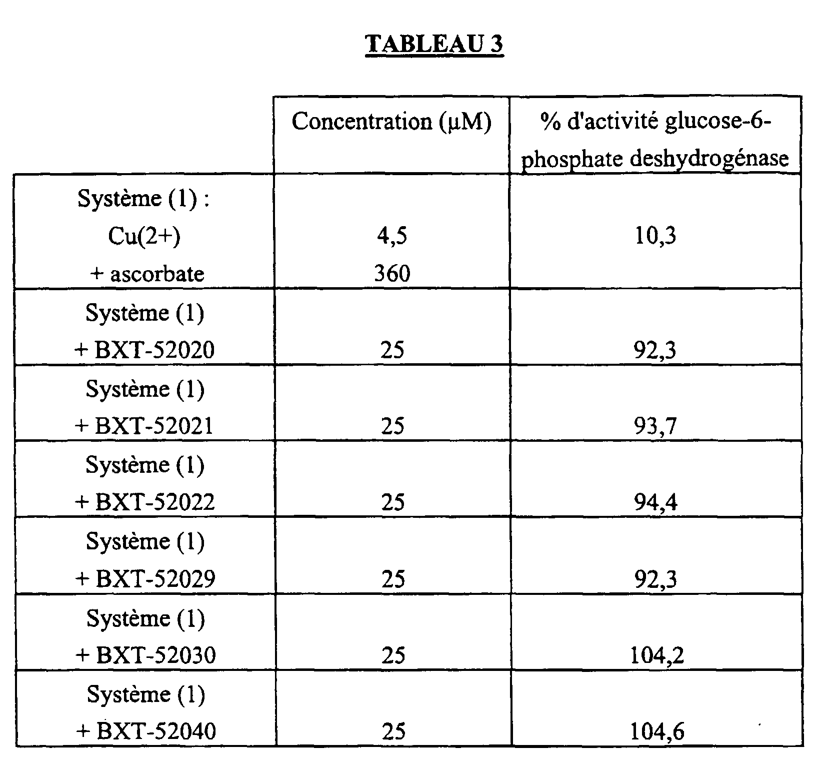

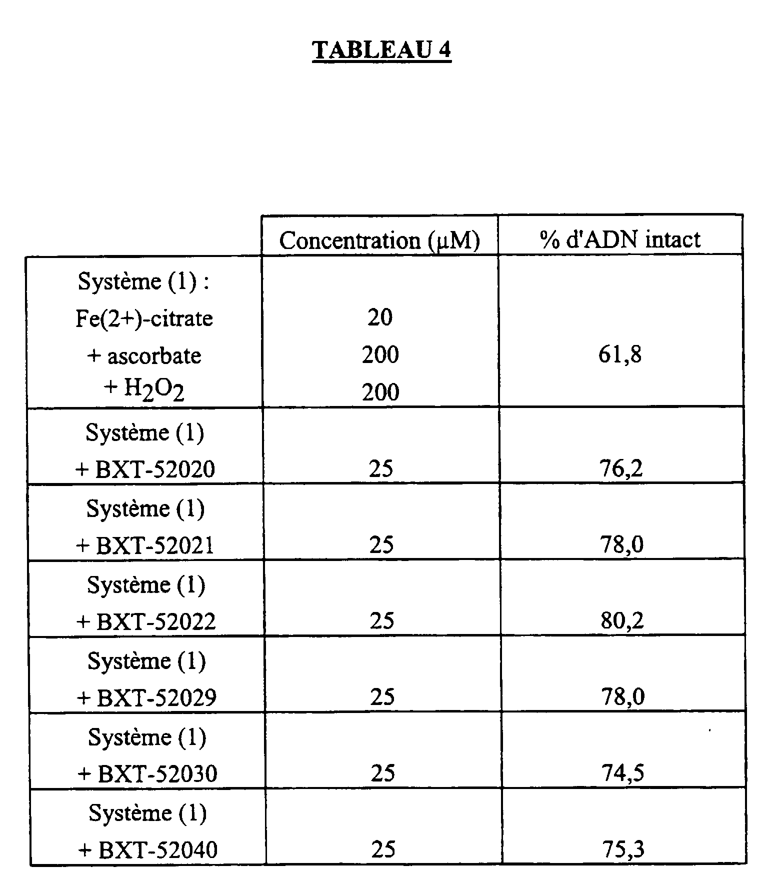

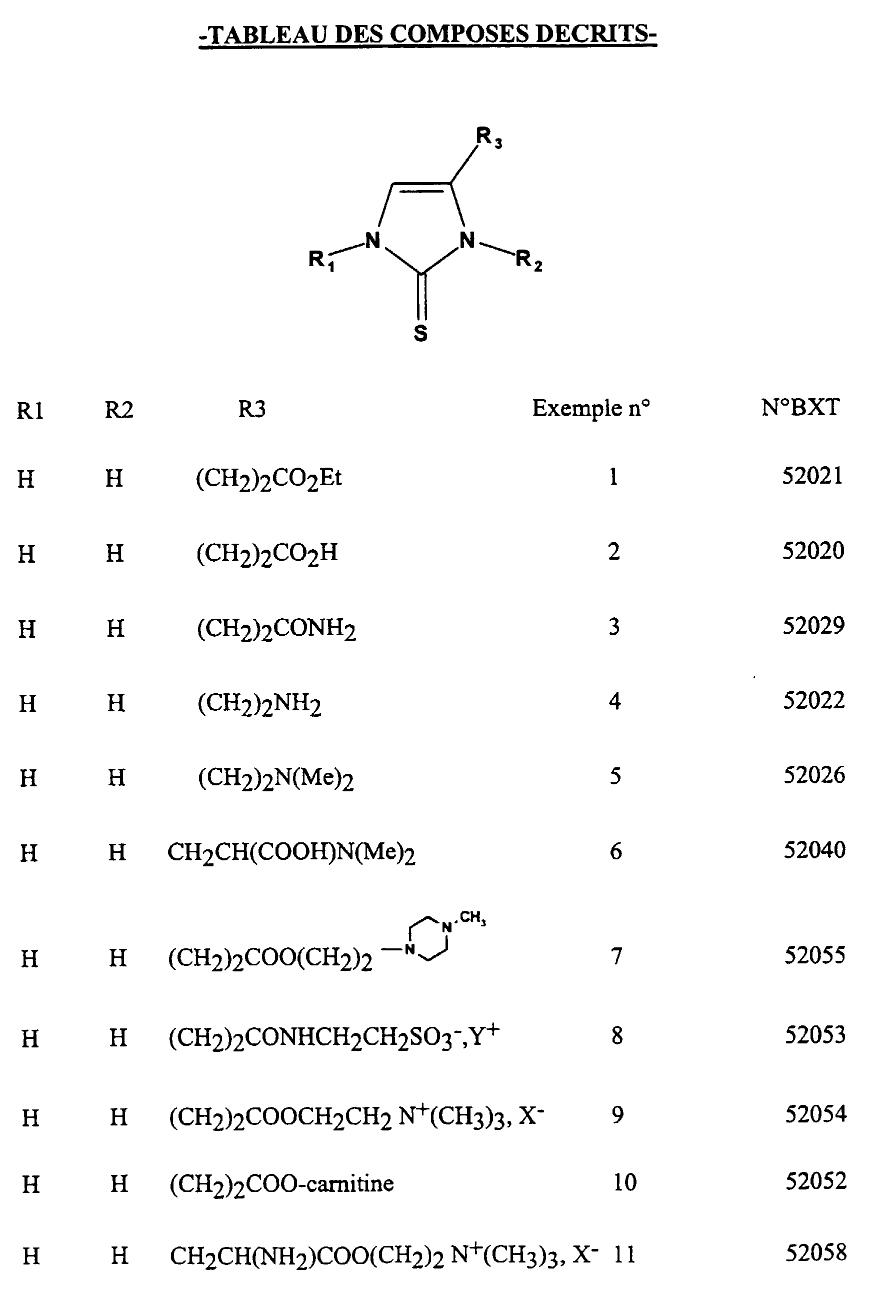

## Revendications

1. Utilisation de dérivés 2-mercapto-imidazole substitués en position 4 (ou 5) de formule (I) générale suivante : 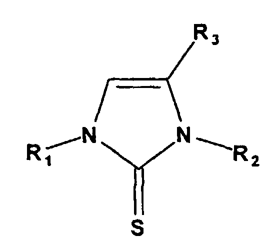 dans laquelle :
R₁ représente l'hydrogène, un alkyle inférieur, en C₁-C₆, un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₂ représente l'hydrogène, un alkyle inférieur, en C₁-C₆ un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
sachant qu'au moins un parmi R₁ et R₂ représente l'hydrogène;
R₃ représente -(CH₂)ₙCOR₄ ; -(CH₂)ₙN+(R₅R₆R₇), X⁻ ; -CH₂CH(COR₄)N⁺_ (R₅R₆R₇), X⁻ ;
R₄ représente :
a) -OR₈ lorsque R₃ représente -CH₂CH(COR₄)N⁺(R₅R₆R₇), X-;-α-aminoacide, de préférence -α-aminoacide naturel; 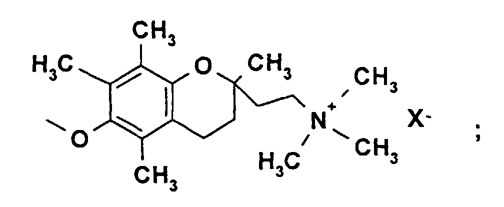
b) lorsque au moins un parmi R₁, R₂ et R₅ à R₈ représente ar(alkyle inférieur en C₁-C₆) ou ar(alkyle inférieur en C₁-C₆) substitué tel que défini, R₄ peut représenter -OR₈ ; -NHR₅ ; -NHCH₂CH₂SO₃⁻,Y⁺; -NHCH₂CH₂CO₂⁻,Y⁺;-OCH₂CH₂N⁺(CH₃)₃, X⁻; 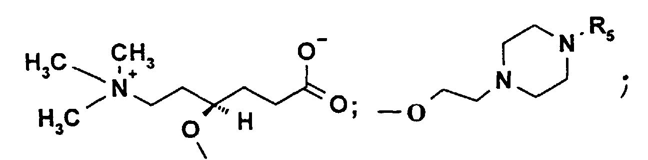
R₅ représente l'hydrogène, un alkyle inférieur en C₁-C₆; un groupement ar(alkyle en C₁-C₆) ou ar(alkyle enC₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₆ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₇ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
R₈ représente l'hydrogène, un alkyle inférieur en C₁-C₆; un groupement ar(alkyle en C₁-C₆) ou ar(alkyle en C₁-C₆) substitué sur la partie aromatique par un ou plusieurs groupements identiques ou différents choisis parmi alkyle inférieur en C₁-C₆, alcoxy inférieure en C₁-C₆, hydroxy, amino, carboxyl en C₁-C₆ ou par un ou plusieurs atomes d'hydrogène;
n= 1 ou 2
X⁻ représente un anion d'un acide cosmétiquement, pharmaceutiquement ou alimentairement acceptable,
Y⁺ représente un cation d'une base cosmétiquement, pharmaceutiquement ou alimentairement acceptable,
comme agents antioxydants.

2. Utilisation selon la revendication 1, pour la fabrication d'une composition pharmaceutique à activité antixoydante, en particulier pour le traitement d'une pathologie impliquant un stress oxydant associée à une surproduction de radicaux libres oxydants et/ou à une décompartimentation intracellulaire du pool de certains métaux de transition tels que le fer, le cuivre ou le manganèse.

3. Utilisation selon la revendication 2, pour la fabrication d'une composition pharmaceutique pour la prévention des altérations tissulaires induites par une ischémie et/ou une reperfusion post-ischémique, et en particulier la prévention d'infarctus du myocarde, et la prévention des arythmies cardiaques post-ischémiques source de fibrillation ventriculaire ; pour la prévention des altérations tissulaires telles qu'oedème, nécroses et fibroses associées à une surproduction de radicaux libres, comprenant notamment le traitement d'intoxications par les xénobiotiques tels que par exemple le paraquat, le diquat, les anthracyclines ou les nitrofurannes ; ou les pathologies associées à un stress oxydant au niveau érythrocytaire, en particulier l'anémie falciforme, la thalassémie, les maladies de déficience en glucose-6-phosphate déshydrogénase et la malaria ; ou pour la protection contre l'irradiation par des rayons ionisants X ou gamma, ainsi que les rayons UV ; ou la protection dans des milieux de conservation de greffons, comme par exemple le coeur, le foie, le rein ou le poumon, en transplantation d'organes.

4. Utilisation selon la revendication 1, pour la fabrication de compositions cosmétiques à activité antioxydante, en particulier pour la protection contre les rayons UV.

5. Utilisation selon la revendication 1, pour la fabrication de compositions alimentaires à activité antioxydante.

6. Utilisation selon l'une des revendications 2 à 5, caractérisée en ce que le 2-mercapto-imidazole précité est présent en une quantité comprise entre 0,1 et 5 % en poids par rapport au poids total de la composition finale, de préférence entre 0,1 et % en poids.

7. Utilisation selon la revendication 2 ou 3, caractérisée en ce que la composition est sous forme de dose unitaire pouvant comprendre de 1 à 500 mg, de dérivé 2-mercapto-imidazole, éventuellement dans un excipient, véhicule ou support pharmaceutiquement acceptable.

8. Composition pharmaceutique, cosmétique ou alimentaire, en particulier à activité antioxydante, caractérisée en ce qu'elle comprend, à titre d'ingrédient actif, au moins un composé 2-mercapto-imidazole de formule générale (I) suivante : 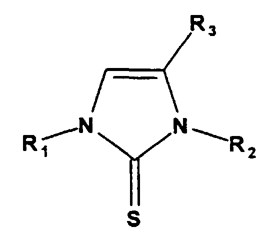 dans laquelle :
R₁ à R₈ sont tels que définis à la revendication 1,
éventuellement dans un excipient, support au véhicule pharmaceutiquement, cosmétiquement ou alimentairement acceptable.

9. Procédé de fabrication de 2-mercapto-imidazole de formule générale (I) suivante : 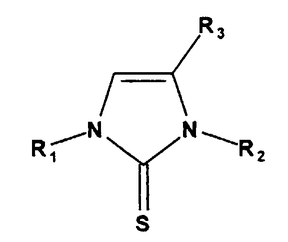 dans laquelle :
R₁ à R₈ sont tels que définis à la revendication 1 de préférence à la condition qu'au moins un parmi R₁ , R₂ , R₅ à R₈ représente un ar(alkyle inférieur en C₁-C₆) ou un ar(alkyle inférieur en C₁-C₆) substitué tel que défini à la revendication 1;
n= 1 ou 2
X⁻ représente un anion d'un acide cosmétiquement, pharmaceutiquement ou alimentairement acceptable,
Y⁺ représente un cation d'une base cosmétiquement, pharmaceutiquement ou alimentairement acceptable,
caractérisé en ce qu'il comprend les étapes essentielles suivantes :
a) préparer ou utiliser un dérivé imidazole substitué en position 4 (ou 5) éventuellement protégé ; si nécessaire optiquement actif ;
b) traiter ce dérivé imidazole par un halogénothionoformiate d'alkyle, d'alkènyle ou d'aryle, en milieu basique dans un solvant polaire ;
c) puis, selon les cas :
i - pour la préparation de composés de formule (I) précédente, dans lesquels R₃ présente les définitions précitées à la condition que R₅, R₆ et R₇ ne sont pas tous simultanément autres que l'hydrogène, hydrolyser en milieu basique, ou en milieu acide en présence d'un piégeur de carbocation ;
ii - pour la préparation des composés de formule (I) précitée dans lesquels R₅, R₆ et R₇ sont simultanément autres que l'hydrogène, protéger le substituant soufré, puis transformer le composé protégé en un composé trialkylammonium et hydrolyser en milieu basique, ou en milieu acide en présence d'un piégeur de carbocation.

10. Procédé selon la revendication 9, caractérisé en ce que le piégeur de carbocation précité est un mercaptan, de préférence un alkyle ou aryle mercaptan, et encore mieux l'acide β-mercaptopropionique.

11. Procédé selon la revendication 9 ou 10, caractérisé en ce que la base précitée est de préférence le bicarbonate de sodium, une amine ou une alkylamine telle que par exemple la diéthylamine ou la triéthylamine.

12. Procédé selon l'une des revendications à 9 à 11, caractérisé en ce que le solvant polaire précité peut être choisi parmi un solvant éthéré comme par exemple l'éther éthylique ou le tétrahydrofuranne, ou un alcool comme par exemple le méthanol.

13. Procédé selon la revendication 9, caractérisé en ce que l'hydrolyse basique précitée est réalisée avec une base inorganique, telle que par exemple la soude ou la lithine, ou organique telle qu'une amine, une alkylamine, en particulier la diéthyle ou la triéthylamine, en particulier en solution dans un solvant polaire, de préférence comprenant un mélange eau/alcool, en particulier le méthanol.

14. Procédé selon la revendication 9 ou 10, caractérisé en ce que l'hydrolyse acide est réalisée à l'aide d'une solution d'acide fort concentré, à pH inférieur à 2, en présence d'un piégeur de carbocation en particulier un mercaptan, de préférence en large excès.

15. Procédé selon l'une des revendications 9 à 14, caractérisé en ce que la protection des substituants soufrés précités est effectuée au moyen d'un halogénoformiate, de préférence un halogénoformiate d'alkyle ou de phényle par exemple le chloroformiate d'éthyle ou de phényle ; et la transformation en composé trialkylammonium est réalisée au moyen d'un agent alkylant comme par exemple un halogénure ou un sulfate d'alkyle, en particulier l'iodure de méthyle, ou le sulfate de diméthyle.

16. Procédé selon l'une des revendications 9 à 15, caractérisé en ce que dans le cadre de la préparation d'un dérivé final optiquement actif, on utilise un composé de départ optiquement actif qui est hydrolysé au moyen d'une solution acide concentrée à pH inférieur à 2 et en présence d'un piégeur de carbocation, de préférence un mercaptan, en large excès.

17. Dérivés 2-mercapto-imidazole substitués en position 4 (ou 5) de la formule (I) générale suivante : 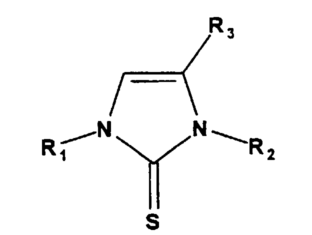 dans laquelle :
R₁, R₂ à R₈ sont tels que définis à la revendication 1 et n=2.
